# EUROPEAN PATENT SPECIFICATION

(11) **EP 0 483 113 B1**
(45) Date of publication and mention of the grant of the patent: **22.09.1999**
(21) Application number: 92101250.6
(22) Date of filing: 26.08.1985
(51) Int. Cl.: C12N 15/53, C12N 9/02

(54) **A method of producing an enzymatically active polypeptide analog of human Cu/Zn SOD**
Methode ein enzymatisch aktives Polypeptidanalog des menschlichen Cu/Zn SOD herzustellen
Méthode de production d'un analogue de Cu/Zn SOD humain enzymatiquement actif

(30) Priority: 27.08.1984 US 644105; 27.08.1984 US 644245; 27.08.1984 US 644551; 27.08.1984 US 644671; 27.08.1984 US 645119
(43) Date of publication of application: 29.04.1992
(62) Divisional of application: 85110683.1
(73) Proprietor: BIO-TECHNOLOGY GENERAL CORPORATION, Iselin, NJ 08830 (US)
(72) Inventor: Hartman, Jacob R., Holon (IL); Kanner, Dov, Rehovot (IL); Bartfeld, Daniel, Nes Ziona (IL)
(74) Representative: Henkel, Feiler, Hänzel

(56) References cited:
- EP-A- 19 474
- EP-A- 138 111

## Description

### Background of the Invention

One aspect of genetic engineering involves the insertion of foreign DNA sequences derived from eucaryotic sources into Escherichia coli or other microorganisms. A further refinement of genetic engineering concerns inducing the resulting microorganism to produce polypeptides encoded by the foreign DNA. Production of polypeptides can be considered a two-step process, with each step including numerous substeps. The two steps are transcription and translation. To produce a polypeptide efficiently and in quantity both steps of the process must be efficient. Transcription is the production of mRNA from the gene (DNA). Translation is the production of polypeptide from the mRNA.

A critical substep of the transcription process is initiation, that is, the binding of RNA polymerase to a promoter-operator region. The sequence of deoxyribonucleotide bases which make up the promoter region may vary and thereby affect the relative efficiency of the promoter. The efficiency depends on the affinity of the RNA polymerase for the promoter.

The efficiency of translation is affected by the stability of the mRNA. Increased stability of the mRNA permits improved translation. Although the exact determinants of mRNA stability are not precisely known, it is known that mRNA secondary structure as determined by the sequence of its bases has a role in stability.

The initial substep of translation involves binding of the ribosome to a base sequence on the mRNA known as the Shine-Dalgarno sequence or the ribosomal binding site (RBS). The synthesis of polypeptides begins when the ribosome migrates along the mRNA to the AUG start codon for translation. Generally these codons are found approximately 10 bases "downstream" from the Shine-Dalgarno site. Factors which increase the efficiency of translation include those which enhance binding of the ribosomes to the Shine-Dalgarno site. It has been shown that the structure of the mRNA in the region of the Shine-Dalgarno sequence and the AUG codon and the distance between the Shine-Dalgarno sequence and the AUG codon each play a critical role in determining the efficiency of translation. Other factors which affect the efficiency of translation are premature termination and attenuation. Efficiency of translation can be improved by removing the attenuation sites.

A difficulty encountered in attempts to produce high amounts of eucaryotic polypeptides in bacterial cells involves the inability of cells producing large amounts of mRNA to grow efficiently. This difficulty can be eliminated by preventing transcription by a process known as repression. In repression, genes are switched off due to the action of a protein inhibitor (repressor protein) which prevents transcription by binding to the operator region. After microorganisms have grown to desired cell densities, the repressed genes are activated by destruction of the repressor or by addition of molecules known as inducers which overcome the effect of the repressor.

Numerous reports may be found in the literature concerning the cloning of eucaryotic genes in plasmids containing the P_{L} promoter from λ bacteriophage. (Bernard, H.V., et al., Gene (1979) 5, 59; Derom, C., et al., Gene (1982) 17, 45; Gheysen, D., et al., Gene (1982) 17, 55; Hedgpeth, J. et al., Mol. Gen. Genet. (1978) 163, 197; Remaut, E., et al., (1981) Gene 15, 81 and Derynck, R., et al., Nature (1980) 287, 193. In addition, European Patent Application No. 041,767, published December 16, 1981, describes expression vectors containing the P_{L} promoter from λ bacteriophage. However, none of these references describe the use of the C_{II} ribosomal binding site.

The use of a vector containing the P_{L} promoter from λ bacteriophage and the C_{II} ribosomal binding site has been described. (Oppenheim, A.B. et al., J. Mol. Biol. (1982) 158, 327 and Shimatake, H. and Rosenberg, M., Nature (1981) 292, 128.) These publications describe the production of increased levels of C_{II} protein but do not involve or describe the production of eucaryotic proteins.

Other vectors which contain the P_{L} promoter and the C_{II} ribosomal binding site have also been described (Courtney, M. et al., PNAS (1984) 81, 669-673; Lautenberger, J.A. et al., Gene (1983) 23, 75-84 and Lautenberger, J.A. et al., Science (1983) 221, 858-860). However, all of these vectors lead to the production of fused proteins which contain the amino terminal portion of the C_{II} protein.

In 1982 Shatzman and Rosenberg presented a poster at the 14th Miami Winter Symposium (Shatzman, A.R. and Rosenberg, M., 14 Miami Winter Symposium, abstract p98 [1982]). This abstract provides a non-enabling disclosure of the use of a vector containing P_{L} from λ bacteriophage, Nut and the C_{II} ribosomal binding site to synthesize a "eucaryotic" polypeptide (SV40 small T antigen is actually not a eucaryotic polypeptide but a viral protein) in an amount greater than 5% of the cell protein in an unnamed bacterial host. The operator used is not defined. Neither an origin of replication nor a gene for a selectable phenotype is identified. This system with which the vector is used is described as including certain host lysogens into which the vector can be stably transformed.

Applicants are aware of the existence of a pending U.S. patent application in the name of M. Rosenberg filed under Serial No. 457,352 by the National Institutes of Health, Dept. of Health and Human Services, U.S.A. Portions of this application have been obtained from the National Technical Information Service, U.S. Dept. of Commerce. However, the claims are not available and are maintained in confidence. The available portions of the application have been reviewed. This disclosure is not enabling. It indicates that the host is important (p8, line 17) but fails to identify any suitable host. It further depends upon the use of a λ mutant which is not specified (p4, line 20). It indicates that the host contains lysogens (p8, line 18) unlike the present invention in which the host is not lysogenic. It mentions cloning and expression of a eucaryotic gene, monkey metaliothionein gene, (p7, line 18) but does not provide details. It specifies that neither the sequence nor the position of any nucleotide in the C_{II} ribosomal binding region has been altered (p3, line 27).

Pending, co-assigned U.S. patent application Serial No. 514,188, filed July 15, 1983, describes novel vectors useful for the expression of polypeptides in bacteria. These vectors include P_{L}O_{L}, N utilization site for binding antiterminator N protein, ribosomal binding site, ATG codon, restriction enzyme site for inserting the gene encoding the desired polypeptide, an origin of replication and a selectable marker. In these vectors the distance between the N utilization site and the ribosomal binding site is greater than about 300 base pairs. In addition, each of these vectors contains a specific ribosomal binding site which cannot be readily replaced. These vectors were not equally useful for expression of different polypeptides.

T₁T₂ rRNA transcription termination sequences have been described (Brosius, J., et al., J. Mol. Biol. 148, 107 (1981)). The placement of T₁T₂ rRNA termination sequences at the 3' end of a procaryotic gene and the expression of such gene under the control of a promoter have been described (Amann, E., et al., Gene (1983) 25, 167; Zabeau, M., et al., The EMBO Journal (1982) 1, 1217).

European patent application no. 81304573.9, published April 14, 1982 under European publication no. 049,619, discloses the use of the λcI857 thermoinducible repressor as a stabilizing element. The repressor is cloned on the plasmid. A λcI90 prophage defective in repressor synthesis is introduced by infection. The prophage is maintained by the cloned repressor at temperatures below 32°C. Any cell losing the plasmid will be lysed. If the temperature is increased to above 38°C, the repressor is destroyed or inactivated and the cells lyse. This stabilization system is not compatible with the vectors of the invention which include λP_{L} promoter and which express polypeptides at temperatures above 38°C.

Origins of replication from constitutive high copy number plasmids are known. For example pOP1Δ6 origin of replication from ColEl has been described (Gelfand, D. H., et al., PNAS (1978) 75, (12), 5869 and Muesing, M., et al. Cell (1981) 45, 235). In addition, high copy number run-away replication plasmids, as distinguished from, constitutive high copy number plasmids, are known (Remant, E., et al. Gene (1983) 22, 103).

The present invention relates to which unexpectedly provide enhanced expression of different polypeptides. By employing different ribosomal binding sites in the vectors of this invention it is possible to achieve enhanced expression levels of different polypeptides relative to the levels achieved with the previous vectors. In addition, using the same ribosomal binding sites as in the previous vectors, it is possible to achieve enhanced expression of the same polypeptides. Moreover, by placing T₁T₂ rRNA termination sequences at the 3' end of the gene encoding a polypeptide whose expression is desired, it is possible to increase the amount of desired polypeptide relative to the total polypeptide produced by a bacterial host. As importantly, the presence of the T₁T₂ rRNA transcription termination sequences permit origins of replication derived from constitutive high copy number plasmids to be incorporated into expression vector without loss of the ability to replicate in constitutive high copy number.

Origin of replication derived from pBR322 or nonconstitutive high copy number plasmids other than runaway high copy number plasmids when incorporated into a vector are capable of producing only a certain number of copies per cell, typically less than 40 copies per cell. By substituting an origin of replication from a constitutive high copy number plasmid it has unexpectedly been found that that level of polypeptide expression is increased.

The preferred vectors of this invention are stabilized in the bacterial host and when bacteria containing plasmids which include the vectors and genes encoding polypeptides are grown, the plasmids are not lost. In this way, yield reduction caused by plasmid instability is overcome. Moreover, use of such preferred vectors avoids the use of antibiotic resistance as a selectable marker, thus permitting lower costs for producing polypeptides.

Superoxide dismutase (SOD) and analogs thereof are some of several polypeptides which may be produced using the novel expression vectors of this invention.

The present invention relates in particular to expression plasmids which unexpectedly provide enhanced expression of superoxide dismutase and analogs thereof using the same ribosomal binding sites as in the previous vectors and by employing different ribosomal binding sites as described in this invention.

The present invention also relates to a method for enhanced production of SOD and analogs thereof in bacteria utilizing these plasmids.

Superoxide dismutase (SOD) and the phenomenon of oxygen free radicals (O₂.⁻) was discovered in 1968 by McCord and Fridovich (McCord, J.M. and Fridovich, I., J. Biol. Chem. 244, 6049-6055 (1969). Superoxide radicals and other highly reactive oxygen species are produced in every respiring cell as by-products of oxidative metabolism, and have been shown to cause extensive damage to a wide variety of macromolecules and cellular components (for review see Fridovich, I. in Advances in Inorganic Biochemistry, eds., Eichhorn, G.L. and Marzilli, L.G. (Elsevier/North Holland, New York), pp. 67-90 (1970) and Freeman, B.A. and Crapo, J.D., Laboratory Investigation 47, 412-426 (1982)). A group of metalloproteins known as superoxide dismutases catalyze the oxidation-reduction reaction 2O₂-^{·} + ₂H⁺ → H₂O₂ + O₂ and thus provide a defense mechanism against oxygen toxicity. There are three known forms of SODs containing different metals in the protein molecules namely iron, manganese or both copper and zinc. All of them catalyze the same reaction with ultimate efficiency, and all operate by a similar mechanism in which the metal is the catalytic factor in the active site. These enzymes fall into several evolutionary groups. The Mn and Fe-SODs are found primarily in prokaryotic cells while CuZn-SOD have been demonstrated in virtually all eucaryotic organisms (Steinman, H.M. in Superoxide Dismutase, ed. Oberley, L.W. (CRC Press, Florida), pp. 11-68 (1982).

Human Cu/Zn SOD-1 is a dimeric metallo-protein composed of identical non-covalently linked subunits, each having a molecular weight of 16000 daltons and containing one atom of copper and one of zinc (Hartz, J.W. and Deutsch, H.F., J. Biol. Chem. 247, 7043-7050 (1972)). Each subunit is composed of 153 amino acids whose sequence was established (Jabusch, J.R., Farb, D.L. Kerschensteiner, D.A. and Deutsch, H.F., Biochemistry 19:2310-2316 (1980) and Barra, D., Martini, F., Bannister, J.V., Schinina, M.W. Rotilio, W.H. Bannister, W.H. and Bossa, F., FEBS Letters 120, 53-56 (1980)). Furthermore, a cDNA clone containing the entire coding region of human SOD-1 was recently isolated and sequenced (Lieman-Hurwitz, J., Dafni, N., Lavie, V. and Groner, Y., Proc. Natl. Acad. Sci. USA 79, 2808-2811 (1982) and Sherman, L., Dafni, N., Leiman-Hurwitz, J. and Groner, Y., Proc. Natl. Acad. Sci. USA 80, 5465-5469 (1983)). EP-A-138 111 describes superoxide dismutase cloning and expression in microorganisms.

The human Cu-Zn SOD analog produced differs from natural human Cu-Zn SOD in that the amino terminus alanine is not acetylated. The natural human SOD is acetylated at the amino terminus alanine (Hartz, J.W. and Deutsch, H.F., J. Biol. Chem. (1972) 247, 7043-7050, Jabusch, J.R., et al., Biochemistry (1980) 19, 2310-2316; Barra, et al., FEBS Letters (1980) 120, 53 and Oberly, L.W. Superoxide Dismutase, Vol. I, CRC Press, Florida, (1982), pp. 32-33). The natural human SOD is likely to be glycosylated like bovine SOD (Huber, W., U.S. Patent No. 3,579,495, issued May 18, 1971). Bacterial-produced human SOD is almost certainly not glycosylated as Escherichia coli does not glycosylate proteins which it produces. The amino acid sequence of the bacterial-produced SOD analog is identical to that of mature human SOD and does not contain a methionine residue at its N-terminus.

This invention provides a method for producing in bacteria and purifying an enzymatically active analog of human Cu/Zn SOD.

Superoxide dismutase is of considerable interest because of its pharmacological properties. Bovine-derived, naturally-occurring superoxide dismutase (orgotein) has been recognized to possess anti-inflammatory properties and is currently marketed in certain European countries, e.g., West Germany, for use in the treatment of inflammation. It is also sold in a number of countries including the United States as a veterinary product for treating inflammation, particularly for treating inflamed tendons in horses.

Additionally, the scientific literature suggests that SOD may be useful in a wide range of clinical applications. These include prevention of oncogenesis and tumor promotion and reduction of cytotoxic and cardiotoxic effects of anti-cancer drugs (Oberley, L.W. and Buettner, G.R., Cancer Research 39, 1141-1149 (1979)); protection of ischemic tissues (McCord, J.M. and Roy, R.S., Can. J. Physiol. Pharma. 60, 1346-1352 (1982)), and protection of spermatozoa (Alvarez, J.G. and Storey, B.T., Biol. Reprod. 28, 1129-1136 (1983)). In addition, there is a great interest in studying the effect of SOD on the aging process (Talmasoff, J.M., Ono, T. and Cutler, R.G., Proc. Natl. Acad. Sci. USA 77, 2777-2782 (1980)).

The present invention also relates to using recombinant human superoxide dismutase (hSOD) or analogs thereof to catalyze the reduction of superoxide radicals in the presence of H⁺, to hydrogen peroxide and molecular oxygen. In particular, the present invention concerns using hSOD analogs to reduce reperfusion injury following ischemia and prolong the survival period of excised isolated organs. It also concerns the use of hSOD or analogs thereof to reduce injury on reperfusion following organ transplantation and spinal cord ischemia.

### Summary of the Invention

This invention concerns a method of producing an enzymatically active polypeptide analog of human Cu/Zn superoxide dismutase having an amino acid sequence substantially identical to that of naturally occurring human Cu/Zn superoxide dismutase, and the biological activity of naturally occurring human Cu/Zn superoxide dismutase which comprises:
(a) growing a culture of bacterial cells, transformed with a plasmid containing DNA encoding said polypeptide analog of human Cu-Zn superoxide dismutase incorporated therein, in a zinc-containing production medium supplemented with a non-growth inhibitory amount of Cu⁺⁺ such that the final concentration each of Cu⁺⁺ and Zn⁺⁺ in the medium is greater than 2 ppm, wherein said transformed cells are capable of expressing the DNA encoding said polypeptide analog of human Cu/Zn supercxide dismutase; and
(b) recovering the enzymatically active polypeptide analog of human Cu/Zn superoxide dismutase so produced.

In a preferred embodiment of this method the bacterial cell is an Escherichia coli cell containing a plasmid which contains a DNA sequence encoding for an analog of human superoxide dismutase.
The invention also concerns a method as claimed in claim 14 of recovering purified enzymatically active eucaryotic superoxide dismutase or an analog thereof produced in the bacterial cell in accordance with the method of this invention. Hereby, the recovering step comprises isolating the bacterial cell from the growth medium and suspending the bacterial cell in a suitable solution having a pH from about 7.0 to about 8.0. The cell wall of the suspended bacterial cell is disrupted and the resulting homogeneous solution is then sonicated under suitable conditions. The sonicated solution is centrifuged under suitable conditions and the supernatant is removed and heated for about 2 hours at about 65°C. The supernatant is then cooled and centrifuged under suitable conditions to produce a clear supernatant protein solution. The resulting supernatant is concentrated to an appropriate volume and subjected to ion exchange chromatography on a suitable anion exchanger equilibrated at a pH from about 7.0 to about 8.0. The resulting solution containing the superoxide dismutase or analog is collected, concentrated to an appropriate volume and dialyzed against a buffered solution with a pH from about 7.0 to about 8.0. This concentrated buffered solution is subjected to ion exchange chromatography on a suitable anion exchanger equilibrated at a pH from about 7.0 to about 8.0 and the anion exchange-protein complex is subjected to a suitable salt gradient. Fractions containing the superoxide dismutase or analog are collected, concentrated and dialyzed against distilled water. The pH of the solution of interest is adjusted to a pH from about 4.0 to about 5.0 with a suitable buffer. The buffered solution is then subjected to ion exchange chromatography with a suitable cation exchanger equilibrated at a pH from about 4.0 to about 5.0. The protein-cation exchanger complex is subjected to a suitable salt gradient and the resulting fractions which contain the purified superoxide dismutase or analog are collected.

### Description of the Figures

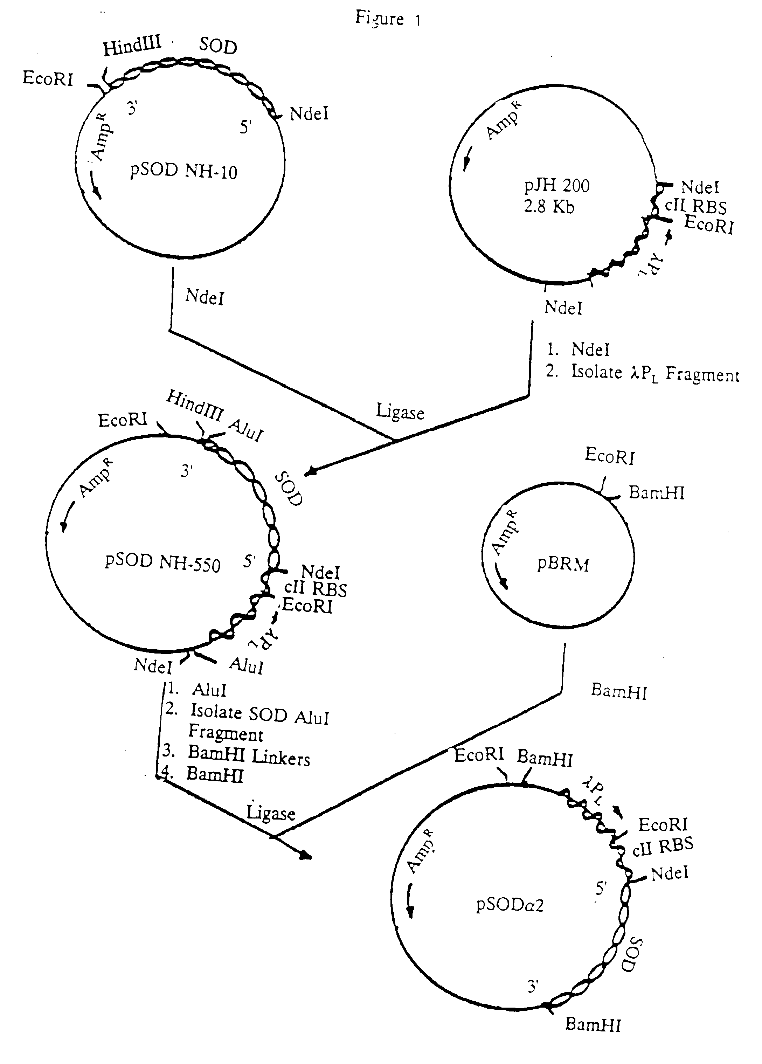

The restriction maps for each of the plasmids shown in Figures 1-5 do not identify all restriction sites present on each plasmid. In some cases restriction sites are shown in one figure but not in another. However, in all cases those restriction sites necessary for a complete understanding of the invention are shown.
Fig. 1. Construction of pSODα2.
   The pJH200 (ATCC No. 39783) expression vector was digested with NdeI. The 550 base pair NdeI fragment containing the λP_{L} promoter and C_{II} ribosomal binding site was isolated and inserted into the unique NdeI site of plasmid pSOD NH-10 which had been digested with NdeI. (Plasmid pSOD NH-10 is derived from a cDNA clone of human SOD [Lieman-Hurwitz, J., et al., PNAS (1982) 79: 2808 ]) The resulting plasmid pSOD NH-550 was digested with AluI. (Only the relevant AluI site is shown in the figure.) The large AluI fragment containing the λP_{L} promoter and the SOD gene was isolated. BamHI linkers were attached and the resulting fragment was digested with BamHI. The BamHI digestion product was inserted into the unique BamHI site of pBRM (ATCC No. 37283) to form pSODα2 (ATCC No. 39786).

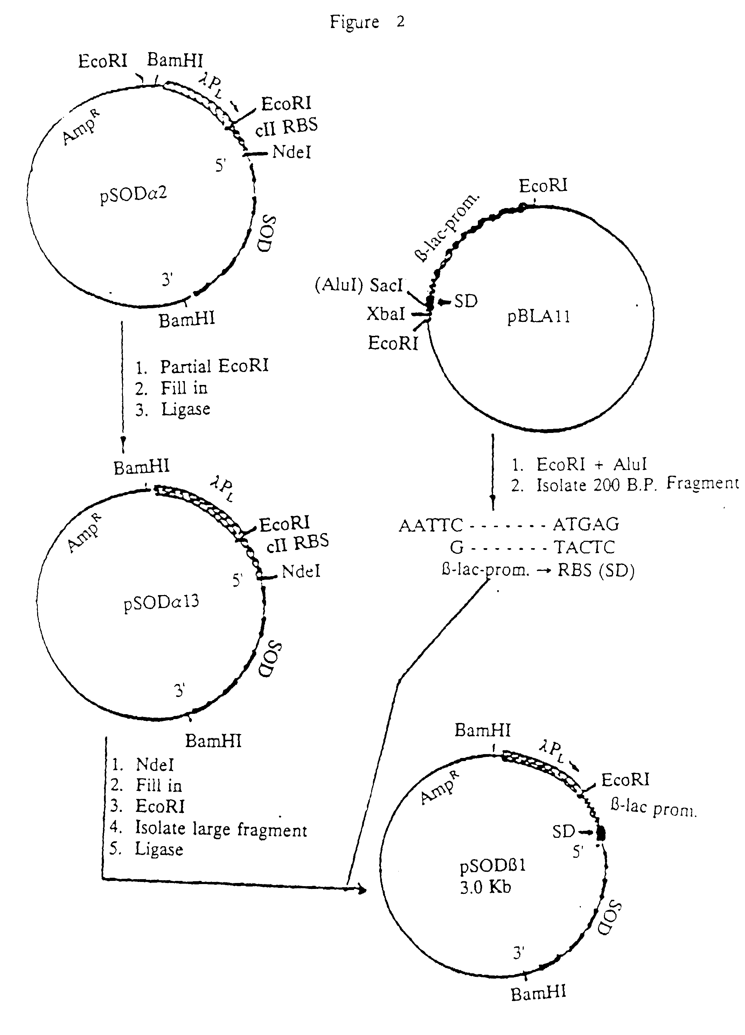

Fig. 2. Construction of pSODα13 and pSODβ1.
   The plasmid pSODα2 (ATCC No. 39786) was partially digested with EcoRI and the resulting linear form DNA was isolated from an agarose gel. The purified DNA was filled in with DNA polymerase I (Klenow) and religated. The resulting clone pSODα13 contains one EcoRI site located at the 5' end of the ribosomal binding site. A fragment containing the β-lactamase promoter and ribosomal binding site was isolated from plasmid pBLAll (ATCC No. 39788) which had been digested with EcoRI and AluI. The 200 base pair fragment was ligated to the large fragment isolated from pSODα13 which had been digested with NdeI, filled in with DNA polymerase I (Klenow) and then digested with EcoRI. The resulting plasmid pSODβ1 contains the ribosomal binding site of the β-lactamase gene and the λP_{L} promoter.

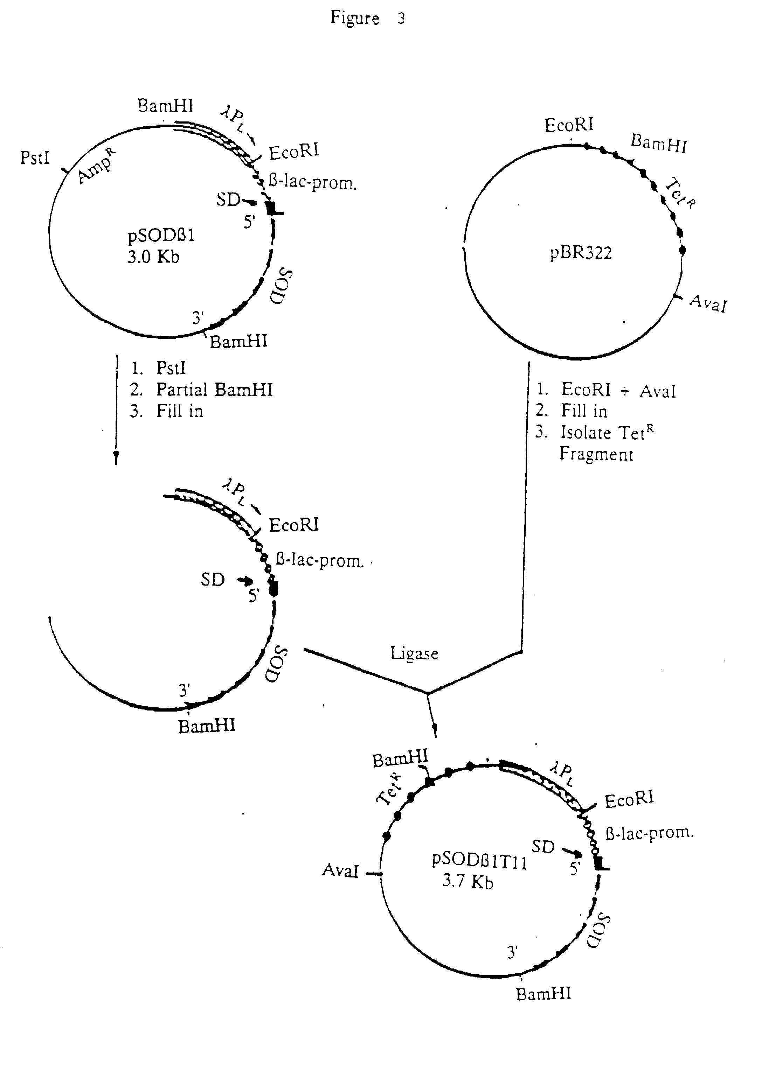

Fig. 3. Construction of pSODβ₁T₁₁.
   Plasmid pBR322 (ATCC No. 37017) was digested with EcoRI and AvaI. The resulting DNA was filled in with DNA polymerase I (Klenow). The Tet^{R} gene fragment was then isolated and ligated to the large fragment isolated from pSODβ1 (Fig. 2) plasmid which had been digested with PstI followed by a partial BamHI digest and then filled in with DNA polymerase I (Klenow). The resulting plasmid pSODβ₁T₁₁ contains the Tet^{R} gene.

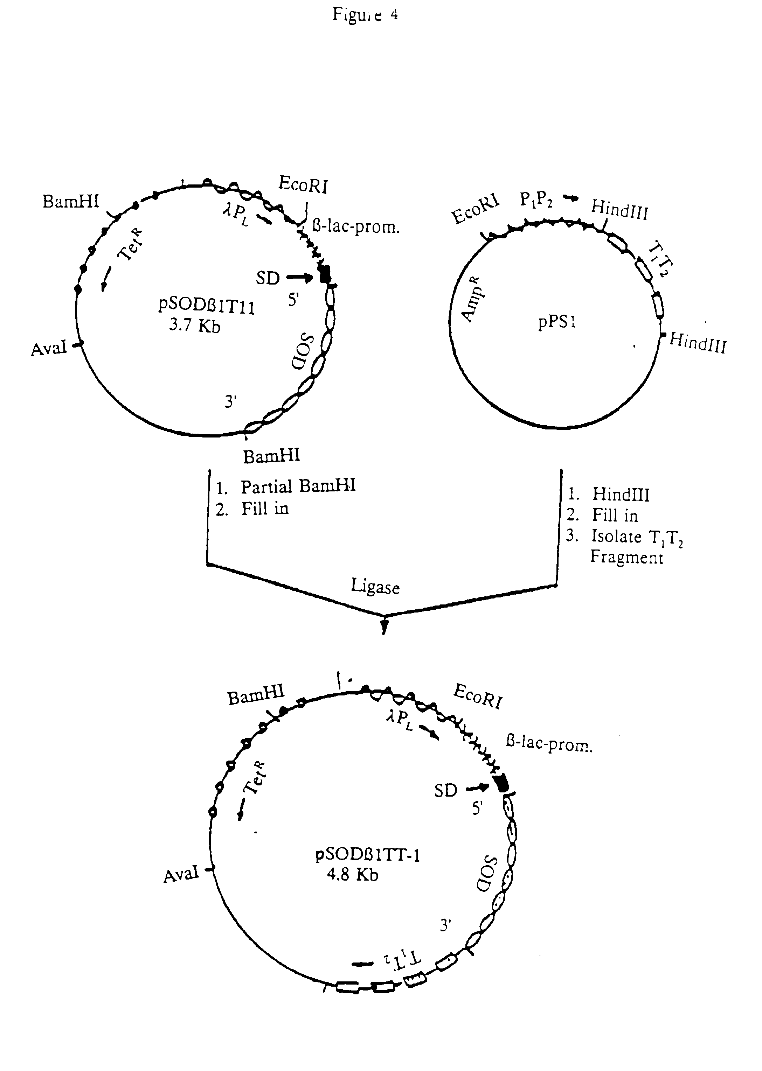

Fig. 4. Construction of pSODβ₁TT-1.
   The rRNA T₁T₂ transcription termination fragment was isolated from plasmid pPS1 (ATCC No. 39807) which had been digested with HindIII and filled in with DNA polymerase I (Klenow). The fragment was ligated to plasmid pSODβ₁T₁₁ (Fig. 3 ) which had been partially digested with BamHI and filled in with DNA polymerase I (Klenow).

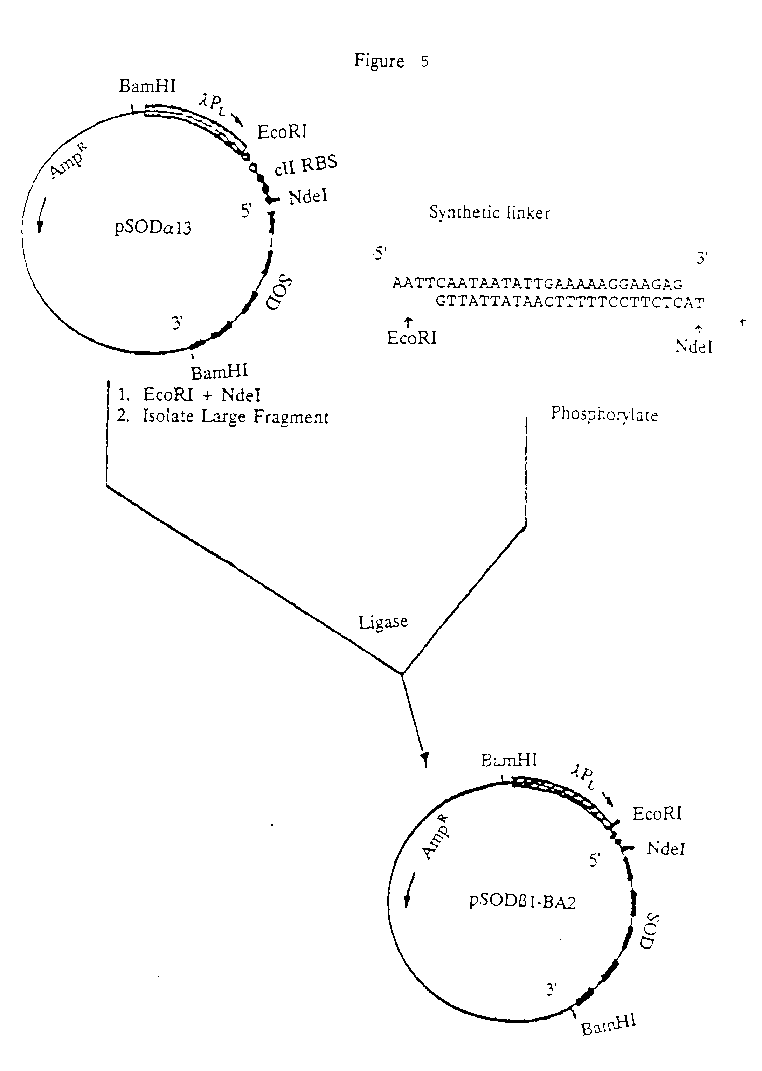

Fig. 5. Construction of pSODβ₁-BA2.
   A synthetic DNA fragment with the sequence: 
5'-AATTCAATAATATTGAAAAAGGAAGAG-3'
GTTATTATAACTTTTTCCTTCTCAT
which is similar to the sequence of the natural β-lactamase ribosomal binding site, was phosphorylated and ligated to the large fragment of pSODα13 plasmid (Fig. 2) which had been digested with NdeI and EcoRI.

### Detailed Description of the Invention

According to the present invention a vector comprising a double-stranded DNA molecule can be used which enables the achievement of enhanced levels of gene expression and polypeptide production.
Upon introduction into a suitable bacterial host cell containing the thermolabile repressor C_{I} the plasmid renders the host cell capable, upon increasing the temperature of the host cell to a temperature at which the repressor is inactivated, of effecting expression of a desired gene inserted into the plasmid and production of a polypeptide encoded by the gene.

The vector includes in 5' to 3' order the following:
a DNA sequence which contains the promoter and operator P_{L}O_{L} from lambda bacteriophage;
the N utilization site for binding antiterminator N protein;
a first restriction enzyme site permitting replacement of the DNA sequence containing the ribosomal binding site which follows thereafter;
a DNA sequence which contains a ribosomal binding site for rendering the mRNA of the desired gene capable of binding to ribosomes within the host cell;
an ATG initiation codon or a DNA sequence which is converted into an ATG initiation codon upon insertion of the desired gene into the vector; and
a second restriction enzyme site for inserting the desired gene into the vector in phase with the ATG initiation codon.

The vector also includes a DNA sequence which contains an origin of replication from a bacterial plasmid capable of automomous replication in the host cell and a selection means selected from the group consisting of DNA sequences which contain a gene associated with a selectable or identifiable phenotypic trait which is manifested when the vector is present in the host cell and DNA sequences which contain the fragment designated cI⁴³⁴, such fragment including the gene for the cI⁴³⁴ repressor protein and its associated promoter and operator sequence. cI⁴³⁴ represses a cI⁴³⁴-lysogen; loss of the plasmid will result in cell lysis. The distance between the 3' end of the P_{L}O_{L} promoter and operator sequence and the 5' end of the N utilization site is less than about 80 base pairs and the distance between the 3' end of the N utilization site and the 5' end of the ribosomal binding site is less than about 300 base pairs.

An optional additional component of the vector is a T₁T₂ rRNA transcription termination sequence located 3' of the second restriction enzyme site. Preferably, the T₁T₂ rRNA transcription termination sequence is less than about 100 base pairs from the 3' end of the second restriction enzyme site. More preferably, the T₁T₂ rRNA transcription termination sequence is less than about 20 base pairs from the 3' end of the second restriction enzyme site.

The vector includes either a DNA sequence containing a gene associated with a selectable or identifiable phenotypic trait which is manifested when the vector is present in the host cell or a DNA sequence containing the cI⁴³⁴ repressor gene which represses a λimm434cI⁻ lysogen or both.

Suitable genes associated with a selectable or identifiable phenotypic trait include those associated with temperature sensitivity or drug resistance, e.g., resistance to ampicillin, chloroamphenicol or tetracycline. When the cI⁴³⁴ repressor gene is contained within a host, the host is prevented from λ imm434cI⁻ prophage induction. Thus, there is no need to use expensive antibiotic selection salines when cI⁴³⁴ is present.

Preferably, the vector includes both a DNA sequence which contains a gene associated with a selectable or identifiable phenotypic trait and a DNA sequence which contains a fragment designated cI⁴³⁴.

Desirably, when the cI⁴³⁴ gene is included on the vector, it is located after the 3' end of the T₁T₂ rRNA sequence.

The vector also includes an origin of replication from a bacterial plasmid capable of autonomous replication in the host cell. Suitable such origins of replication may be obtained from a number of sources, e.g., from pBR322 or pR1.

Preferably, the vector has an origin of replication from a constitutive high copy number bacterial plasmid capable of autonomous replication in the host cell of at least 400 constitutive copies of the vector. More preferably, this origin of replication is from Co1E1. Most preferably, the origin is from plasmid pOP1Δ6.

Another component of the vector is a first restriction enzyme site permitting replacement of the DNA sequence containing the ribosomal binding site which follows thereafter. Numerous such sites may be used. Suitable sites include EcoRI.

Another component of the vector is a second restriction enzyme site for insertion of the desired gene into the plasmid in phase with the ATG initiation codon. Numerous such sites may be used. Suitable sites include NdeI, ClaI, HindIII, SmaI, BglII, XbaI, SacI and AluI.

Generally it is desirable that the second restriction enzyme site also functions as the second restriction site necessary to permit replacement of the DNA sequence containing the ribosomal binding site. If the second restriction site is not also used for this purpose then the vector usable in this invention must also include a third restriction enzyme site after the ribosomal binding site but prior to the second restriction site.

Preferably, the vector contains two unique restriction enzyme sites. The first site permits replacement of the DNA sequence containing the ribosomal binding site. The second site permits insertion of the desired gene into the plasmid in phase with the ATG initiation codon. The term "unique restriction enzyme" site as employed herein means a restriction enzyme site which occurs only once in the plasmid. In a presently preferred embodiment, EcoRI is the first restriction enzyme site and NdeI is the second restriction enzyme site.

Preferably, the vector is a covalently closed circular double-stranded molecule. However, it is not essential that the plasmid be covalently closed.

The plasmid achieves its enhanced expression levels after the host cell is heated to a temperature at which the C_{I} repressor protein is inactivated. A temperature above about 38°C is effective for this purpose and since it is desired that unnecessary heat damage to the host cells be avoided to as great an extent as possible, it is generally desirable that the temperature not exceed 42°C by more than a few degrees.

One important component of the vector is the ribosomal binding site. Suitable sites are C_{II} from lambda bacteriophage having the sequence: 
TAAGGAAATACTTACAT
ATTCCTTTATGAATGTA;
 a mutant of C_{II} from lambda bacteriophage having the sequence: 
TAAGGAAGTACTTACAT
ATTCCTTCATGAATGTA;
 the major head protein gene of bacteriophage lambda having the sequence: 
TTTTTTTACGGGATTTTTTTATG
AAAAAAATGCCCTAAAAAAATAC;
 the natural β -lactamase ribosomal binding site derived from pBR322;
a synthetic oligonucleotide having the sequence: 
AATTCGAGCGCAAGGAAACAGGCTCA
    GCTCGCGTTCCTTTGTCCGAGTAT;
 a synthetic oligonucleotide having the sequence: 
AATTCAATAATATTGAAAAAGGAAGAG
GTTATTATAACTTTTTCCTTCTCAT;
 and a natural ribosomal binding site derived from Bacillus thurengensis.

Relative to vectors described previously, the vectors usable in this invention may be used to obtain enhanced expression of a wide variety of genes encoding desirable polypeptide products. Suitable genes include those encoding growth hormones, e.g., bovine, porcine, chicken or human growth hormones; superoxide dismutase; apolipoprotein E or analogs of any of the preceding. By analog is meant a polypeptide having the same activity as the naturally occurring polypeptide but having one or more different amino acids added or deleted, or both, at the N-terminus of the polypeptide. However, SOD analogs described have an amino acid sequence identical to that of mature human SOD.
The preferred host for use with the plasmids usable in this invention is Escherichia coli. The presently preferred strains are A1637, A1645, A2602, A2097, A1563 and A1645 (λi434cI⁻ mini Tn10).

A1645 is presently the most preferred strain for expression of superoxide dismutase or an analog thereof.

A1645 and A1645 (λ i434cI⁻ mini Tn10) are presently the more preferred strains for expression of animal growth hormone genes.

A2097 is presently the most preferred strain for the expression of the gene which produces:
1) an analog of bGH having the amino acid sequence met-asp-gln added to the amino-terminus of the phenylalanine form of authentic bGH; or
2) an analog of cGH having the amino acid methionine added to the amino-terminus of the phenylalanine form of natural cGH.

A1637 was obtained from C600 by inserting transposon containing tetracycline resistance gene within the galactose operon as well as the lambda system for expression which is close to galactose operon. C600 is available from the American Type Culture Collection, as ATCC Accession No. 23724.

A1645 was obtained from A1637 by selection for Gal⁺ (ability to ferment galactose) as well as loss of tetracycline resistance. It still contains the lambda expression system but part of the transposon has been removed by selection. Its phenotype is C600 r⁻ m⁺ gal⁺ thr⁻ leu⁻ lacZ⁻ bl (λc1857 ΔH1Δ BamH1 N⁺). A1645 has been deposited with the American Type Culture Collection in Rockville, Maryland, U.S.A. containing various plasmids as described more fully hereinafter.

A1645 (λi434cI⁻ mini Tn10) was derived by infecting Escherichia coli strain A1645 containing a plasmid with imm434 cI3008 mini Tn10Δ16Δ17 at 30°C. Tetracycline resistant colonies were isolated and purified. This strain containing plasmid pHG50 has been deposited with the American Type Culture Collection under ATCC Accession No. 39805.

A2602 and A1563 are derived from SA500. Their phenotypes are SA500 his⁻ ile⁻ gal⁺ Δ 8 (λcI857 ΔH1 Δ Bam N⁺) and SA500 his⁻ ile⁻ gal⁺ Δ8 lacZ⁻ A21 (λcI857 int2 xis1 nutL₃Δ H1), respectively. A2097 is derived from A1645. Its phenotype is A1645 lacΔχA21 proC::Tn10.

Prototrophic strains of Escherichia coli which enable high level polypeptide expression even when grown in a minimal media may also be used as hosts for the vectors usable in this invention. Preferred prototrophic strains include A4200 and A4255. Strain A4255 containing the plasmid p9200 has been deposited with the ATCC under Accession No. 53215. Even more preferred are biotin independent prototrophic strains such as A4346 containing the plasmid pHG44 which has been deposited with the ATCC under Accession No. 53218.

Lytic strains of Escherichia coli may also be used as hosts for the vectors usable in this invention. Suitable lytic strains include those which produce, at the temperature at which the polypeptide is produced but at a rate slower than that at which the polypeptide is produced, a substance e.g., an enzyme like endolysin which will cause the cell to lyse. This permits the cell to produce relatively large amounts of the desired polypeptide before the amount of the lysing substance produced reaches the level which causes cell lysis. Examples of suitable lytic strains include those containing the P1cI^{ts} plasmid such as strain A4048 containing pHG44 which has been deposited with the ATCC under Accession No. 53217.

All deposits were made pursuant to the Budapest Treaty on the International Recognition of the Deposit of microorganisms except that pBR322 and pBRM are fully available from the American Type Culture Collection as ATCC Accession Nos. 37017 and 37283, respectively, and D4 was deposited under ATCC Accession No. 31826 in connection with the filing of a U.S. patent application.

The vector may be formed by methods well known to those of ordinary skill in the art to which the invention relates. Such methods are described in greater detail in various publications identified herein, the contents of which are hereby incorporated by reference into the present disclosure in order to provide complete information concerning the state of the art.

One presently preferred vector is pJH200. This vector was introduced into Escherichia coli strain A1645 using a conventional transformation method. The resulting host vector system has been deposited under ATCC Accession No. 39783. A gene encoding a desired polypeptide, e.g. bovine growth hormone, may be inserted into pJH200.

A second preferred vector, pR0211, was constructed from a partial NdeI digest of pJH200. Bovine growth hormone cDNA has been inserted into pR0211 by digesting the vector with NdeI and HindIII, isolating the large fragment and ligating to it bGH cDNA obtained from pAL500 (ATCC Accession No. 39782). The resulting plasmid is designated pR012.

Plasmid pR012 has been partially digested with PvuII followed by NdeI. A synthetic DNA fragment coding for the first 24 amino acids of the N-terminus of authentic bGH has been ligated to the digested pRO12. The resulting plasmid is designated pSAL 5200-6.

The vectors usable in this invention may also be engineered to yield plasmids which produce human superoxide dismutase (SOD) or analogs thereof. A fragment of pJH200 (ATCC Accession No. 39783) containing the λP_{L} promoter and C_{II} ribosomal binding site was isolated and then inserted into a plasmid pSOD NH-10 which contains the gene for human SOD to form a plasmid designated pSOD NH-550 as shown in Fig. 1. A fragment containing both the λP_{L} promoter and the SOD gene was isolated from pSOD NH-550 following digestion with AluI. After the addition of BamHI linkers and subsequent restriction with BamHI, the fragment was inserted into the unique BamHI site of pBRM. pBRM is a high copy number plasmid which has been deposited under ATCC Accession No. 37283. The resulting plasmid is designated pSODα2. It has the restriction map shown is Fig. 1. This plasmid has been deposited in E. coli strain A2097 under ATCC Accession No. 39786.

Plasmid pSODα2 (ATCC Accession No. 39786) contains the C_{II} ribosomal binding site. This ribosomal binding site has been replaced with a fragment containing the β-lactamase promoter and Shine-Dalgarno ribosomal binding site isolated from an EcoRI-AluI digest of pBLA11. (Plasmid pBLAll has the restriction map shown in Fig. 2 and has been deposited in Escherichia coli strain A1645 under ATCC Accession No. 39788.) The C_{II} ribosomal binding site is removed from plasmid pSODα2 as shown in Fig. 2. pSODα2 is partially restricted with EcoRI, filled in with DNA polymerase I (Klenow) and religated, so that the only remaining EcoRI site in the plasmid is located at the 5' end of the C_{II} RBS. The resulting plasmid, designated pSOD 13 was digested with NdeI, filled in with DNA polymerase I (Klenow) and then digested with EcoRI. The large fragment was isolated and ligated to the fragment containing the β-lactamase promoter and ribosomal binding site isolated from pBLA11 to form plasmid pSODβ1.

pSODβ1 may be modified to include a tetracycline resistance gene fragment (Tet^{R}) instead of an ampicillin resistance gene fragment (Amp^{R}) The Amp^{R} fragment was removed from pSOD 1 by digestion with PstI followed by partial BamHI. The resulting plasmid was filled in with DNA, polymerase I (Klenow). The Tet^{R} gene fragment was separately isolated from an EcoRI-AvaI digest of pBR322, filled in and ligated to the filled in plasmid. (Plasmid pBR322 is widely available, e.g. from the American Type Culture Collection as ATCC Accession No. 37017). The then resulting plasmid is designated pSODβ₁T₁₁. It has the restriction map shown in Fig. 3.

One further plasmid which may be used to produce human superoxide dismutase is designated pSODβ1-BA2. Its construction from pSODα13 is shown in Fig. 5.

The vectors usable in this invention may also be engineered to yield plasmids which produce an analog of human Cu-Zn superoxide dismutase (SOD) which differs from natural human SOD in that the amino terminus is not acetylated. Such a plasmid has been constructed according to Fig. 4 and has been designated pSODβ₁TT-1.

Various host vector systems involve E. coli A1637, A1645, A2606, A2097 or A1563 if the plasmid does not contain the cI⁴³⁴ fragment and strain A1645 ( i434cI⁻ mini Tn10) if the plasmid contains the cI⁴³⁴ fragment. Host vector systems of this invention also involve prototrophic E. coli such as A4200, A4255 and include biotin independent host vector systems such as A4346. Lytic host vector systems of this invention include those wherein the host is A4048, particularly A3111.

The host vector systems and the plasmid described herein may be used to produce different polypeptides such as bovine, porcine, chicken and human growth hormones, human superoxide dismutase and human apoliprotein E. To do so, the host vector system is grown under suitable conditions permitting production of polypeptide which is then recovered.

Suitable conditions involve growth of the host vector system for an appropriate period of time at about 42°C. Desirably, the period of growth at 42°C for all host vector systems except those designed to produce human polipoprotein E is about 1 to 5 hours. The period of growth for host vector systems designed to produce human apolipoprotein E is desirably about 15 minutes. Suitable media include casein hydrolysate.

By means of the preceding method a number of bGH, pGH, cGH, hGH, ApoE and SOD analogs have been prepared.

SOD analogs have been prepared which have an amino acid sequence identical to that of natural SOD except or variations at the N-terminus. Examples include the following:
1) natural human SOD which is non-acetylated; and
2) natural human SOD which is non-acelylated and non-glycosylated.

These SOD analogs may be used to catalyze the dismutation or univalent reduction of the superoxide anion in the presence of proton to form hydrogen peroxide as shown in the following equation:

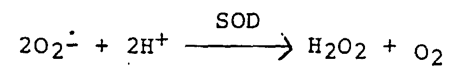

Veterinary and pharmaceutical compositions may also be prepared which contain effective amounts of SOD or one or more SOD analogs and a suitable carrier. Such carriers are well-known to those skilled in the art. The SOD or analog may be administered directly or in the form of a composition to the animal or human subject, e.g., to treat a subject afflicted by inflammations or to reduce injury to the subject by oxygen-free radicals on reperfusion following global ischemia or organ transplantation e.g., kidney transplantation. The SOD or analog may also be added directly or in the form of a composition to the perfusion medium of an isolated organ, e.g., to reduce injury to an isolated organ by oxygen-free radicals on perfusion after excision, thus prolonging the survival period of the organ, e.g. cornea. Additionally, the SOD or analog may be used to reduce neurological injury.

A method of producing enzymatically active eucaryotic superoxide dismutase (SOD) or an analog thereof in a bacterial cell has been discovered. The bacterial cell contains and is capable of expressing a DNA sequence encoding the superoxide dismutase or analog. The method comprises maintaining the bacterial cell under suitable conditions and in a suitable production medium. The production medium is supplemented with an amount of Cu⁺⁺ so that the concentration each of Cu⁺⁺ and Zn⁺⁺ in the medium is greater than about 2 ppm.

The bacterial cell can be any bacterium in which a DNA sequence encoding eucaryotic superoxide dismutase has been introduced by recombinant DNA techniques. The bacterium must be capable of expressing the DNA sequence and producing the protein product. The suitable conditions and production medium will vary according to the species and strain of bacterium.

The bacterial cell may contain the DNA sequence encoding the superoxide dismutase or analog in the body of a vector DNA molecule such as a plasmid. The vector or plasmid is constructed by recombinant DNA techniques to have the sequence encoding the SOD incorporated at a suitable position in the molecule.

In a preferred embodiment of the invention the bacterial cell is an Escherichia coli cell. The preferred strain of E. coli is strain A1645. The E. coli cell of this invention contains a plasmid which encodes for the SOD or its analog. In a preferred embodiment of the invention the SOD is human superoxide dismutase or an analog thereof.

The preferred embodiments of the invention concern E. coli strains which contain the plasmids pSOD β₁, pSOD α2, pSODβ T11, pSOD β₁-BA2 or pSOD β₁TT1. Methods of constructing these plasmids are described in the Description of the Figures and the plasmids themselves are described in Example 3. These plasmids can be constructed from available starting materials by persons of ordinary skill in the art. E. coli strains containing the various plasmids which are useful in constructing plasmids encoding eucaryotic superoxide dismutase have been deposited with the American Type Culture Collection, Rockville, Maryland 20852, pursuant to the provisions of the Budapest Treaty on the International Recognition of the Deposit of Microorganisms for the purposes of Patent Procedure. These plasmids and their ATCC accession numbers are: pBRM ATCC 37283; pSODα2 ATCC 39786; pBR322 ATCC 37017; pBLAll ATCC 39788; pJH200 ATCC 39783 and pPSI ATCC 39807.

In a specific embodiment of the invention an enzymatically active human superoxide dismutase analog is produced by an E. coli strain A2097 cell containing the plasmid psODα2. This cell has been deposited with the American Type Culture Collection under accession number ATCC 39786.

The suitable production medium for the bacterial cell can be any type of acceptable growth medium such as casein hydrolysate or LB (Luria Broth) medium. Suitable growth conditions will vary with the strain of E. coli and the plasmid it contains, for example E. coli A1645 containing plasmid pSOD β₁T11 are induced at 42°C and maintained at that temperature from about 1 to about 5 hours. The suitable conditions of temperature, time, agitation and aeration for growing the inoculum and for growing the culture to a desired density before the production phase as well as for maintaining the culture in the production period are described in Example 26.

The concentration of Cu⁺⁺ ion in the medium that is necessary to produce enzymatically active SOD will vary with the type of medium used. In a casein hydrolysate medium the Cu⁺⁺ ion concentration of 200 ppm has been found effective.

In a LB medium a Cu⁺⁺ concentration of 75 ppm has been found effective. It is preferred that in all complex types of growth mediums the concentration of Cu⁺⁺ in the medium is from about 50 to about 250 ppm.

Most eucaryotic superoxide dismutases are Cu/Zn metalloproteins. In a specific embodiment of the invention Zn⁺⁺ is added as a supplement to the medium, so that the concentration of Zn⁺⁺ in the medium is greater than about 2 ppm. In a preferred embodiment of the invention Cu⁺⁺ and Zn⁺⁺ concentrations are supplemented by adding 0.8 g/l of CuSO₄•5H₂O and 10 mg./l of ZnSO₄•7H₂O.

The specific ingredients of the suitable stock, culture, inoculating and production mediums may vary and are known to those of ordinary skill in the art. Specific examples of suitable mediums are described in Example 26.

The invention also concerns a method of recovering purified enzymatically active eucaryotic SOD or an analog thereof produced in a bacterial cell in accordance with the methods of this invention.

The bacterial cell is first isolated from the production medium after the culture has been chilled. The cell may be isolated by any nondisruptive method such as centrifugation or filtration. The cell is then suspended in a suitable solution having a pH from about 7.0 to about 8.0. It is preferred that a 50mM sodium phospate solution of a pH of about 7.8 be used. The cell wall is disrupted by a suitable means such as a blender or a cell disrupter and the resulting homogeneous suspension is sonicated under suitable conditions. The sonication may be by means of a continuous flow cell. The resulting sonicated solution is then centrifuged under suitable conditions so as to separate the cell debris from the protein supernatant solution.

The supernatant is then heated for about 2 hours at about 65°C, cooled and centrifuged under the same conditions as were used in the previous centrifugation step in order to result in a clear protein solution as a supernatant. The supernatant is then concentrated to an appropriate volume, e.g. concentrated to 1 liter in an ultrafiltration device using a 10,000 molecular weight cutoff.

The concentrated protein solution is then subjected to ion exchange chromatography on a suitable anion exchanger equilibrated at a pH from about 7.0 to about 8.0. It is preferred that the chromatography should be carried out on a DEAE-Sephacel column equilibriated with 150mM sodium phosphate buffer having a pH of about 7.8. The resulting flow through solution containing the superoxide dismutase or analog is then collected, concentrated to an appropriate volume and dialyzed against a buffered solution with a pH from about 7.0 to about 8.0. This can be done in an ultrafiltration device against a 20mM Tris-HCl solution of a pH of about 7.8.

This concentrated solution is then subjected to ion exchange chromatography on a suitable anion exchanger equilibrated at a pH from about 7.0 to about 8.0. A QAE-Sepharose column equilibrated with 20mM Tris-HCl having a pH of about 7.8 is suitable. The protein bound to the anion exchanger is subjected to a suitable salt gradient e.g. 0-200mM NaCl in 20mM Tris HCl pH 7.8. The resulting fractions containing the SOD or analog are collected, concentrated e.g. with an ultrafiltration device, dialyzed against distilled water and the adjusted to a pH from about 4.0 to about 5.0 with a suitable buffer. In a preferred embodiment the solution of interest is brought to 100mM Sodium Acetate by adding 1M Sodium Acetate having a pH of about 4.8.

This buffered solution is again subjected to ion exchange chromatography but with a cation exchanger. A CM-Sepharose column equilibrated with a 100mM sodium acetate buffer having pH of about 4.7 is suitable. The protein bound to the cation exchanger is subjected to a suitable salt gradient, such as 100 to 500mM NaCl in 100mM sodium acetate pH 4.7 and the resulting fractions containing the purified SOD or analog are collected.

The fractions containing the purified SOD may be concentrated e.g. by ultrafiltration and lyophilized for storage.

### Examples

The examples which follow are set forth to aid in understanding the invention but are not intended to, and should not be construed to, limit its scope in any way. The examples do not include detailed descriptions for conventional methods employed in the construction of vectors, the insertion of genes encoding polypeptides of interest into such vectors or the introduction of the resulting plasmids into bacterial hosts. Such methods are well-known to those of ordinary skill in the art and are described in numerous publications including by way of example the following:
T. Maniatis, E.F. Fritsch and J. Sambrook, Molecular Cloning; A Laboratory Manual, Cold Spring Harbor Laboratory, New York (1982).
Methods in Enzymology, vol. 65, "Nucleic Acids (Part 1)," edited by Lawrence Grossman and Kivie Moldave, Academic Press, New York (1980).
Methods in Enzymology, vol. 68, "Recombinant DNA," edited by Ray Wu, Academic Press, New York (1981).
Methods in Enzymology, vol. 100, "Recombinant DNA (Part B)," edited by Ray Wu, Lawrence Grossman and Kivie Moldave, Academic Press, New York (1983).
Methods in Enzymology, vol. 101, "Recombinant DNA (Part C)," edited by Ray Wu, Lawrence Grossman and Kivie Moldave, Academic Press, New York (1983).
Principles of Gene Manipulation, An Introduction to Genetic Engineering, 2nd Edition, edited by R.W. Old and S.B. Primrose, University of California Press (1981).
H.V. Bernard, et al., Gene (1979) 5, 59.
A.B. Oppenheim, et al., J. Mol. Biol. (1982) 158, 327.
E. Remaut, et al., Gene (1981) 15, 81.

### EXAMPLE 1

### Expression Vectors

As used herein the term "expression vector" refers to a group of plasmids useful for expressing desired genes in bacteria, particularly in E. coli. The desired gene may be inserted into the expression vector or alternatively, the promoters on the expression vector may be excised and placed in front of the desired gene.

### pJH200

pJH200 is composed of a DNA inserted into the multicopy plasmid pBR322. The salient features of the λDNA are that it contains the λP_{L} promoter, the leftward N utilization site (nut_{L}), an EcoRI restriction site, the t_{RI} termination site, followed by the C_{II} ribosomal binding site and an ATG initiation codon which is part of the NdeI restriction site. One hundred and sixteen (116) base pairs downstream of the NdeI restriction site are four unique restriction sites. The restriction sites enable facile insertion of the desired gene. The C_{II} ribosomal binding site differs from the natural ribosomal binding site by a single point mutation.

pJH200 was constructed from pOG11 (A. Oppenheim, et al., J. Mol. Biol. (1982) 158; 327) and contains the λP_{L} promoter and the C_{II} ribosomal binding site found in pOG11. However, 346 bp of λDNA located between the λP_{L} promoter and the C_{II} ribosomal binding site have been deleted, and an EcoRI restriction site has been introduced at the junction between these two elements. Also, a multi-restriction site linker was introduced "downstream" of the ribosome binding site. pJH200 has been deposited with the American Type Culture Collection under ATCC No. 39783.

### pRO211

pRO211 was derived from pJH200 by eliminating one of the two NdeI restriction sites.

pJH200, pRO211 and derivatives thereof containing eucaryotic genes may be maintained in suitable E. coli hosts. The most important feature of a suitable host is that it provide the thermosensitive repressor cI857 and the antitermination N protein. (M.E. Gottesman, et al., J. Mol. Biol. (1980) 140; 57-75).

pRO211 has numerous advantages over previously described expression vectors including:
1. extremely high levels of expression
   The vector is capable of directing expression of foreign proteins in E. coli at levels as high as 35% of the total cellular protein.
2. replaceable ribosomal binding site
   pRO211 contains a unique EcoRI site which is located "upstream" of the ribosomal binding site, and an NdeI site located "downstream" of the ribosomal binding site. Thus, the ribosomal binding site is bounded by two unique restriction sites. This enables facile excision of the present ribosomal binding site (the λC_{II} ribosomal binding site) and substitution of virtually any other natural or synthetic ribosomal binding site without altering other features of the plasmid. This greatly facilitates optimal expression of desired polypeptides.
3. thermoinducible regulation of expression
   The λP_{L} promoter is inactive when the C_{I} repressor is bound to it. The cI857 repressor is thermosensitive, that is, it binds to the promoter at 30°C but is inactivated at 42°C. Thus, by increasing the temperature of fermentation to 42°C the host bacteria are induced to produce the desired protein.
   The advantages of such a system include the following:
   (a) A foreign protein which is toxic to Escherichia coli can be produced late in the fermentation process thus avoiding early cell death,
   (b) Overproduction of a protein may stabilize the protein and prevent proteolytic degradation. (Cheng, Y.E., et al., Gene (1981) 14, 121). Thus, "instantaneous" overproduction using a tightly regulated promoter such as λP_{L} may be preferable to continuous low level production.
4. simplified induction protocol
   Protein production by the plasmids described in this patent application and in copending, coassigned U.S. patent application Serial No. 514,188 is regulated by the thermosensitive cI857 repressor.
   The induction protocol required by the plasmids described in the copending, coassigned application involved induction at 42°C followed by growth at 38°C. In contrast, the optimal induction of protein synthesis when using the vectors pJH200, pRO211 or their plasmid derivatives involved induction at 42°C followed by growth at the same temperature, i.e. 42°C. This eliminates the need to cool the fermentor.
5. copy number
   The λP_{L} promoter in pJH200 and pRO211 is found on a plasmid with a copy number higher than the λ transducing phage vectors which are present in E. coli. This increases expression levels.
6. ribosome binding site and initiation codon
   This expression vector contains a strong procaryotic ribosomal binding site (RBS) as well as a translation initiation codon (ATG). Thus, any eucaryotic gene may be cloned without adding the initiation codon. Furthermore, the efficient RBS increases levels of expression. The ribosome binding site is the λC_{II} ribosomal binding site. The sequence of the ribosomal binding site is: 
TAAGGAAGTACTTACAT
ATTCCTTCATGAATGTA
   One base pair is different from the ribosomal binding site found in the wild type λ.
7. convenient restriction site
   The expression vector has a unique NdeI restriction site which contains within the site the ATG initiation codon. This permits proper positioning of the desired gene. The unique NdeI site is found immediately after the ribosomal binding site.
8. convenient restriction sites for gene insertion
   Located 116 base pairs downstream of the NdeI restriction site are 4 other unique restriction sites in the following order: BglII, SmaI, HindIII and ClaI. The multiplicity of unique restriction sites enables facile insertion of desired genes.
9. nut site
   N protein, which is provided by the host, binds the Nut site on the expression vector and thereby prevents termination of transcription at the t_{RI} site or premature transcription termination within the cloned gene.

### Strains

Suitable hosts for the described vectors and plasmids are strains of E. coli suitable for transformation, including A1637, A2602, A1563, A1645 (c600 r⁻m⁺ ga1⁺ thr⁻ leu⁻ lac⁻ b1 (λcI857ΔH1 ΔBamHI N⁺)) and A2097 (A1645 lac ΔχA21 proC::Tn 10).

### EXAMPLE 2

### Human Cu-Zn Superoxide Dismutase (SOD)

The starting point for Cu-Zn SOD cDNA modifications is the plasmid pS61-10 described in Lieman-Hurwitz, J., et al., PNAS (1982), 79: 2808. The SOD cDNA is also described in copending U.S. patent application Serial No. 489,786, filed April 29, 1983. The SOD cDNA was modified to introduce an NdeI restriction site at the 5' end of the gene and a HindIII restriction site at the 3' end of the gene. The resulting plasmid, pSOD NH-10, contains SOD cDNA bounded by unique restriction sites.

### I. pSODα2

The construction of pSODα2 is shown in Fig. 1 and described in the Description of the Figures. pSODα2 has been deposited with the American Type Culture Collection under ATCC No. 39786. To construct pSODα2, the λP_{L} promoter, the Nut_{L} and the C_{II} ribosomal binding site were excised from the expression vector pJH200 and placed in front of the SOD gene of plasmid pSOD NH-10. Then, the fragment containing both the promoter, the RBS and the SOD gene was inserted into the vector pBRM (Hartman,J.R.,et al., PNAS 79: 233-237 (1982). pBRM has been deposited with the American Type Culture Collection under ATCC No. 37283.

pSODα2 was introduced into Escherichia coli strain A2097 by transformation using known methods. The clones obtained produce upon growth and induction an SOD analog protein. The amount of SOD analog produced by pSODα2 was about 0.1 - 0.3% of the total protein produced by the bacteria as calculated from scanning of Coomasie-stained SDS polyacrylamide gels (Table II). The SOD analog produced is probably identical to that produced by pSODβ1 described in the following paragraph.

### II. pSODβ1

The construction of pSODβ1 is shown in Fig. 2 and described in the Description of the Figures. To construct pSODβ1, the C_{II} RBS of pSODα2 was replaced with the β-lactamase promoter and RBS derived from pBLAll. pBLAll has been deposited with the American Type Culture Collection under ATCC No. 39788.

pBLAll contains the promoter and ribosomal binding site of the β-lactamase gene found in pBR322 between coordinates 4157 and 4353. An EcoRI linker was added upstream of the promoter and a multi-restriction site linker was added immediately after the initiation codon ATG. Thus, the sequence of the coding strand beginning with the initiation codon is ATGAGCTCTAGAATTC.

pSODβ1 was introduced into Escherichia coli strain A1645 by transformation using known methods. The clones obtained produce upon growth and induction an SOD analog. The human Cu-Zn SOD analog produced differs from natural human Cu-Zn SOD in that the amino terminus alanine is not acetylated, as demonstrated by amino acid sequencing stoichiometry while the natural human SOD is acetylated at the amino terminus alanine (Hartz, J.W. and Deutsch, H.F., J. Biol. Chem. (1972) 234:7043-7050; Jabusch, J.R., et al., Biochemistry (1980) 19:2310-2316; Barra, et al., FEBS Letters (1980) 120:53 and Oberley, L.W., Superoxide Dismutase, Vol. I, (1982), CRC Press, Florida, pp. 32-33.). Furthermore, the natural human SOD is glycosylated (Huber, W., U.S. Patent No. 3,579,495, issued May 18, 1971) while bacterial-produced human SOD is almost certainly not glycosylated, because Escherichia coli does not glycosylate proteins which it produces. The amino acid sequence of the bacterial-produced SOD analog is identical to that of mature human SOD and does not contain a methionine residue at its N-terminus.

The amount of SOD produced by pSODβ1 was about 3-8% of the total protein produced by the bacteria as calculated from scanning of Coomasie-stained SDS polyacrylamide gels (Table II). The methods used to grow the strain, recover the SOD produced and purify the SOD are the same as those described hereinafter in Example 7 for pSOD β₁T₁₁·

### III. pSODβ₁T₁₁

The construction of pSODβ₁T₁₁ is shown in Fig. 3 and described in the Description of the Figures. The β-lactamase gene of pSODβ1 was replaced with the gene coding for tetracycline resistance derived from pBR322.

The amount of SOD analog produced by pSODβ₁T₁₁ was about 8-13% of the total protein produced by the bacteria as calculated from scanning of Coomasie-stained SDS polyacrylamide gels (Table II). The SOD analog produced is identical to that produced by pSODβ1.

### IV. pSODβ₁-BA2

The construction of pSODβ₁-BA2 is shown in Fig. 5 and described in the Description of the Figures. The C_{II} ribosomal binding site of pSODα13 was replaced by a synthetic DNA fragment with the sequence: 
AATTCAATAATATTGAAAAAGGAAGAG
GTTATTATAACTTTTTCCTTCTCAT
 which is similar to the sequence of the natural β-lactamase _{RBS.}

pSODβ₁-BA2 was introduced into Escherichia coli strain A1645 by transformation using methods known to those of ordinary skill in the art. The clones obtained produce upon growth and induction an analog of human SOD. The amount of SOD produced by pSODβ₁-BA2 was about 2 - 4% of the total protein produced by the bacteria as calculated from scanning of Coomasie-stained SDS polyacrylamide gel (Table II). The SOD analog produced is identical to that produced by pSODβ1.

**TABLE II**

| Plasmid | RBS | Host | %SOD³ | Remarks |
|---|---|---|---|---|
| pSODα2 | C_{II} | A2097 | 0.1-0.3 | Amp^{R} |
| pSODβ₁ | BLA¹ | A1645 | 3-8 | Amp^{R} |
| pSODβ₁T₁₁ | BLA¹ | A1645 | 8-13 | Tet^{R} |
| pSODβ₁TT-1 | BLA¹ | A1645 | 10-15 | Tet^{R};T₁T₂ |
| pSODβ₁-BA2 | BLA² | A1645 | 2-4 | Amp^{R} |

| | | | | |
|---|---|---|---|---|
| 1. Promoter and ribomosal binding site of β-lactamase gene. | | | | |
| 2. Synthetic ribosomal binding site corresponding to that of the β-lactamase gene. | | | | |
| 3. Amount of SOD analog produced expressed as per centage of total bacterial protein. | | | | |

### ABBREVIATIONS

| | |
|---|---|
| Amp^{R} = | Ampicillin resistance |
| | |
| Tet^{R} = | Tetracycline resistance |
| | |
| T₁T₂ = | Transcription termination sequences |

### EXAMPLE 3

### Growth Of SODβ₁T₁₁

### 1. Stock Cultures

Stock cultures of pSODβ₁T₁₁ were grown on casein medium (see Inoculum), then diluted two-fold with freezing medium and stored at -80°C. Freezing medium contains per 500 ml:

| | |
|---|---|
| K₂HPO₄ | 6.3 gr |
| KH₂PO₄ | 1.8 gr |
| Na Citrate | 0.45 gr |
| MgSO₄·7H₂O | 0.09 gr |
| (NH₄)₂SO₄ | 0.9 gr |
| Glycerol | 44.0 gr |

### II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was innoculated with 2 - 10% innoculum and incubated 15 hours at 30°C, pH 7±0.5 with agitation and aeration to maintain a dissolved oxygen level above 20% air saturation.

### III. Production

The production medium contains:

| | |
|---|---|
| Casein hydrolysate | 20 g/l |
| Yeast extract | 10 g/l |
| K₂HPO₄ | 2.5 g/l |
| MgSO₄·7H₂O | 1 g/l |
| NaCl | 5 g/l |
| Biotin | 0.1 mg/l |
| Thiamine | 1 mg/l |
| Trace elements solution | 3 ml/l |
| CuSO₄ | 0.8 g/l |
| ZnSO₄ | 10 mg/l |

The medium also contains 12.5 mg/liter tetracycline. The tetracycline is optional for production, but is always found in the medium used for growing the inoculum.

Biotin, thiamine and tetracycline in concentrated solution were filter sterilized separately and added to the sterile production medium before inoculation. Sterile glucose solution was added initially to supply 10 g/l. At the induction step another 10 g/l of glucose was added.

The trace elements solution contains:

| | |
|---|---|
| FeCl₃ | 16 g/l |
| ZnCl₂·4H₂O | 2 g/l |
| CoCl₂·6H₂O | 2 gf/l |
| Na₂MoO₄·2H₂O | 2 g/l |
| CaCl₂·2H₂O | 1 g/l |
| CuCl₂ | 1 g/l |
| H₃BO₃ | 0.5 g/l |
| Conc. HCl | 100 ml/l |

The medium is inoculated with 0.5 - 10% inoculum culture and incubated at 30°C. Agitation-aeration rates are set to maintain a dissolved oxygen level above 20% air saturation. The pH is maintained at 7±0.2 with NH₃. Once cell concentration reaches about 3.5 g/l (OD₆₆₀ = 10) induction is started.

The temperature is raised to 42°C and maintained at 42°C for 1 - 5 hours. The culture is then chilled and cells are recovered by centrifugation for enzyme purification.

### Recovery Of SOD

One and one half kilograms of bacterial cells (wet cake) are suspended in 12 liters of 50 mM sodium phosphate (pH 7.8), in a Polytron (Kinematica) blender while controlling the speed to minimize foaming. The homogeneous suspension is continuously passed through a Dynomill cell disrupter KD5 (Willy, A. Bachofen, Basel). The homogeneous suspension of disrupted cells is sonicated using a continuous flow cell and centrifuged in a CEPA 101 centrifuge. The supernatant is heated for 2 hours at 65°C, cooled and centrifuged as before. The clear supernatant is concentrated to 1 liter in a Millipore Pellicon ultrafiltration device using 10,000 molecular weight cutoff cassettes (type PTGC). The concentrated protein solution is passed through a DEAE-Sepharose column (2 Kg DEAE Sepharose) equilibrated with 150 mM sodium phosphate buffer (pH 7.8). The flow through solution is collected, concentrated and dialyzed in a Pellicon ultrafiltration device against 20 mM Tris-HCl, pH 7.8, and then applied on to a QAE-Sepharose column equilibrated with 20 mM Tris-HCl buffer. The column is developed with a 20 mM Tris HCl buffer, pH 7.8, and a salt gradient (0 - 200 mM NaCl). SOD-containing fractions are collected, concentrated using a Pellicon ultrafiltration device, dialzed against distilled water and then brought to 100 mM sodium acetate by adding 1 M sodium acetate buffer, pH 4.8. The protein solution is then further separated on a CM-Sepharose column equilibrated with 100mM sodium acetate buffer, pH 4.7. The column is developed using the same buffer and a salt gradient (100-500mM NaCl). SOD containing fractions are collectea, concentrated using a Pellicon ultrafilter device and lyophilized.

### EXAMPLE 4

### Activity Of SOD Produced By pSODβ₁T₁₁

The enzymatic activity of the SOD analog produced by pSODβ₁T₁₁ prepared in Example 7 was assayed by monitoring the inhibition of reduction of ferricytochrome-c as described by McCord and Fridovich, J. Biol. Chem. (1969), 244: 6049-6055. The results demonstrated that the activity of pSODβ₁T₁₁-produced SOD analog was comparable to that of natural human SOD (Sigma) and to that of bovine SOD (Orgotein: Grunenthal GMBH).

### EXAMPLE 5

### Expression Vectors

### I. p579

The vector p579 is composed of a P_{L} promoter, and N utilization site (Nut_{L}), the C_{II} ribosomal binding site bounded by unique EcoRI and NdeI restriction sites, an ATG initiation codon and the T₁T₂ transcription termination signals derived from the end of the rrnB ribosomal RNA gene operon of Escherichia coli. These elements are cloned on pBR322 carrying the ampicillin resistance gene.

p579 was prepared by inserting the T₁T₂ transcription termination signals contained on the plasmid pPS1 into the HindIII site of the vector pRO211. pPS1 has been described in Sarmientos, et al., Cell (1983) 32, 1337-1346 and has been deposited with the American Type Culture Collection under ATCC Number 39807. p579 and its derivatives containing eucaryotic genes may be maintained in suitable Escherichia coli hosts. The most important feature of the host is that it provides the thermosensitive repressor cI857 and the antitermination N protein. (Gottesman, M. et al., J. Mol. Biol. (1980) 140, 57-75).

p579 has numerous advantages over previously described expression vectors including:
1. extremely high levels of expressions
   This vector is capable of directing expression of foreign proteins in E. coli at levels as high as 42% of the total cellular protein. This level of expression is higher than that described for other similar λP_{L} plasmids lacking the T₁T₂ transcription termination sequences.
2. transcription termination signals
   The vector p579 contains the T₁T₂ transcription termination signals placed "downstream" from the λP_{L} promoter and C_{II} ribosomal binding site. The high levels of expression which are obtained when using this vector, are due in part to the presence of the T₁T₂ transcription terminators at the end of the inserted gene, as the T₁T₂ transcription terminators are capable of terminating transcription of N modified RNA polymerase. Thus the transcription terminators prevent the λP_{L} controlled transcription of undesired plasmid proteins, thereby enhancing the relative yields of the desired protein.
3. replaceable ribosomal binding sites
   p579 contains a unique EcoRI site which is located "upstream" of the ribosomal binding site, and a unique NdeI site located at the ATG initiation codon. Thus, the ribosomal binding site is bounded by two unique restriction sites. This enables facile excision of the present ribosomal binding site (the λC_{II} ribosomal binding site) and substitution of virtually any other natural or synthetic ribosomal binding site without altering other features of the plasmid. This greatly facilitates optimal expression of desired polypeptides.
4. thermoinducible regulation of expression
   The λP_{L} promoter is inactive when the C_{I} repressor is bound to it. The cI857 repressor is thermosensitive, that is, it binds to the promoter at 30°C but is inactivated at 42°C. Thus, by increasing the temperature of fermentation to 42°C the host bacteria are induced to produce the desired protein.
   The advantages of such a system include the following:
   (a) a foreign protein which is toxic to Escherichia coli can be produced late in the fermentation process thus avoiding early cell death.
   (b) overproduction of a protein may stabilize the protein and prevent proteolytic degradation (Cheng, Y.E., et al., Gene (1981) 14, 121). Thus, "instantaneous" overproduction using a tightly regulated promoter such as λP_{L} may be preferable to continuous low level production.
5. simplified induction protocol
   The plasmids derived from p579 are induced at about 42°C and maintained at 42°C throughout the period of protein synthesis. The induction protocol for plasmids derived from pMG100 and pND5 described in copending, coassigned U.S. Patent Application Serial No. 514,188 requires a temperature shift to 42° C followed by an extended period of growth at 38°C. The optimal induction protocol for p579 does not require the cooling step to 38°C and is thus simplified.
6. high copy number
   The λP_{L} promoter in p579 is found on a plasmid with a copy number higher than that of λ transducing phage vectors which are used in Escherichia coli. This increases expression levels.
7. ribosome binding site and initiation codon
   This expression vector contains a strong procaryotic ribosomal binding site (RBS) as well as a translation initiation codon (ATG). Thus, any eucaryotic gene may be cloned without adding an initiation codon. Furthermore, the efficient RBS increases levels of expression. The ribosome binding site is the λC_{II} ribosomal binding site which we previously cloned into the vector pND5. The sequence of the ribosomal binding site is 
TAAGGAAGTACTTACAT
ATTCCTTCATGAATGTA.
 One base pair is different from the ribosomal binding site found in wild type λ.
8. convenient restriction site
   The expression vector has a unique NdeI restriction site which contains within it, the ATG initiation codon. This permits proper positioning of the desired gene. The unique NdeI site is found immediately after the ribosomal binding site.
9. convenient restriction sites for gene insertion
   Located 116 base pairs downstream of the NdeI restriction site are unique restriction sites BglII and SmaI, in that order. These unique restriction sites enable facile insertion of desired genes.
10. nut site
   N protein, which is provided by the host, binds to the Nut site on the expression vector and thereby prevents termination of transcription at the t_{RI} site or premature transcription termination within the cloned gene.

### Strains

Suitable hosts for the described vectors and plasmids are strains of Escherichia coli suitable for transformation, including A1637, A2602, A1563, A1645 (c600 r⁻m⁺ gal⁺ thr⁻ leu⁻ lac⁻bl (λc1857 ΔH1 ΔBamHI N⁺) and A2097 (A1645 lac ΔχA21 proC::Tn 10).

### EXAMPLE 6

### Human Cu-Zn Superoxide Dismutase (SOD)

### I. pSODβ₁TT-1

The construction of pSODβ₁TT-1 is shown in Fig. 4 and described in the Description of the Figures. The plasmid pSODβ₁TT-1 was obtained by insertion of the T₁T₂ termination sequences at the 3' end of the SOD gene found in pSODβ₁T₁₁ (Fig. 15).

The plasmid pSODβ₁TT-1 was introcuced into Escherichia coli strain A1645 by transformation using methods known to those of ordinary skill in the art. This strain produced upon growth an SOD analog. The amount of SOD analog produced was about 10 - 15% of the total protein produced by the bacteria as calculated by scanning Coomasie-stained SDS polyacrylamide gels.

The methods used to grow the strain, recover the SOD analog produced and purify the SOD analog, are described in Example 15.

The level of expression of pSODβ₁TT-1 was higher than that obtained from pSODβ₁T₁₁ (Table II) due to the T₁T₂-induced reduction in transcription and translation of non-desired DNA sequences.

The human Cu-Zn SOD analog produced differs from natural human Cu-Zn SOD in that the amino terminus alanine is not acetylated, as demonstrated by amino acid sequencing stoichiometry. The natural human SOD is acetylated at the amino terminus alanine (Hartz, J.W. and Deutsch, H.F., J. Biol. Chem. (1972) 247, 7043-7050, Jabusch, J.R., et al., Biochemistry (1980) 19, 2310-2316; Barra, et al., FEBS Letters (1980) 120, 53 and Oberley, L.W., Superoxide Dismutase, Vol. I, CRC Press, Florida, (1982), pp. 32-33). The natural human SOD is glycosylated (Huber, W., U.S. Patent No. 3,579,495, issued May 18, 1971). Bacterial-produced human SOD is almost certainly not glycosylated as Escherichia coli does not glycosylate proteins which it produces. The amino acid sequence of the bacterial-produced SOD analog is identical to that of mature human SOD and does not contain a methionine residue at its N-terminus.

### EXAMPLE 7

### Growth of pSODβ₁TT-1

### 1. Stock Cultures

Stock cultures of pSODβ₁T₁₁ were grown on casein medium (see Inoculum), then diluted two-fold with freezing medium and stored at -80°C. Freezing medium contains per 500 ml:

| | |
|---|---|
| K₂HPO₄ | 6.3 g |
| KH₂PO₄ | 1.8 g |
| Na Citrate | 0.45 g |
| MgSO₄·7H₂O | 0.09 g |
| (NH₄)₂SO₄ | 0.9 g |
| Glycerol | 44.0 g |

### II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was inoculated with 2 - 10% innoculum and incubated 15 hours at 30°C, pH 7±0.5 with agitation and aeration to maintain a dissolved oxygen level above 20% air saturation.

### III. Production

The production medium contains:

| | |
|---|---|
| Casein hydrolysate | 20 g/l |
| Yeast extract | 10 g/l |
| K₂HPO₄ | 2.5 g/l |
| MgSO₄·7H₂O | 1 g/l |
| NaCl | 5 g/l |
| Biotin | 0.1 mg/l |
| Thiamine | 1 mg/l |
| Trace elements solution | 3 ml/l |
| CuSO₄ | 0.8 g/l |
| ZnSO₄ | 10 mg/l |

The medium also contains 12.5 mg/liter tetracycline. The tetracycline is optional for production, but is always found in the medium used for growing the inoculum.

Biotin, thiamine and tetracycline in concentrated solution were filter sterilized separately and added to the sterile production medium before inoculation. Sterile glucose solution was added initially to supply 10 g/l. At the induction step another 10 g/l of glucose was added.

The trace elements solution contains:

| | |
|---|---|
| FeCl₃ | 16 g/l |
| ZnCl₂·4H₂O | 2 g/l |
| CoCl₂·6H₂O | 2 g/l |
| Na₂MoO₄·2H₂O | 2 g/l |
| CaCl₂·2H₂O | 1 g/1 |
| CuCl₂ | 1 g/l |
| H₃BO₃ | 0.5 g/l |
| Conc. HCl | 100 ml/l |

The medium is inoculated with 0.5 - 10% inoculum culture and incubated at 30°C. Agitation-aeration rates are set to maintain a dissolved oxygen level above 20% air saturation. The pH is maintained at 7±0.2 with NH₃. Once cell concentration reaches about 3.5 g/l (OD₆₆₀ = 10) induction is started.

The temperature is raised to 42°C and maintained at 42°C for 1 - 5 hours. The culture is then chilled and cells are recovered by centrifugation for enzyme purification.

### Recovery Of SOD

One and half kilograms of bacterial cells (wet cake) are suspended in 12 liters of 50 mM sodium phosphate (pH 7.8), in a Polytron (Kinematica) blender while controlling the speed to minimize foaming. The homogeneous suspension is continuously passed through a Dynomill cell disrupter KD5 (Willy,'A. Bachofen, Basel). The homogeneous suspension of disrupted cells is sonicated using a continuous flow cell and centrifuged in a CEPA 101 centrifuge. The supernatant is heated for 2 hours at 65°C, cooled and centrifuged as before. The clear supernatant is concentrated to 1 liter in a Millipore Pellicon ultrafiltration device using 10,000 molecular weight cutoff cassettes (type PTGC). The concentrated protein solution is passed through a DEAE-Sepharose column (2 Kg DEAE Sepharose) equilibrated with 150 mM sodium phosphate buffer (pH 7.8). The flow through solution is collected, concentrated and dialyzed in a Pellicon ultrafiltration device against 20 mM Tris-HCl, pH 7.8, and then applied on to a QAE-Sepharose column equilibrated with 20 mM Tris-HCl buffer. The column is developed with a 20 mM Tris HCl buffer, pH 7.8, and a salt gradient (0 - 200 mM NaCl). SOD-containing fractions are collected, concentrated using a Pellicon ultrafiltration device, dialzed against distilled water and then brought to 100 mM sodium acetate by adding 1 M sodium acetate buffer, pH 4.8. The protein solution is then further separated on a CM-Sepharose column equilibrated with 100mM sodium acetate buffer, pH 4.7. The column is developed using the same buffer and a salt gradient (100-500mM NaCl). SOD containing fractions are collected, concentrated using a Pellicon ultrafilter device and lyophilized.

### EXAMPLE 8

### Activity Of SOD Produced By pSODβ₁TT-1

The enzymatic activity of the SOD analog produced by pSODβ₁TT-1 prepared in Example 15 was assayed by monitoring the inhibition of reduction of ferricytochrome-c as described by McCord and Fridovich, J. Biol. Chem. (1969), 244, 6049-6055. The results demonstrated that the activity of pSODβ₁TT-1-produced SOD analog was comparable to that of natural human SOD and to that of bovine SOD (Orgotein: Grunenthal GMBH).

### EXAMPLE 9

The yield and activity of human superoxide dismutase produced by the SOD host-vector systems described in Example 3 may be improved by modifying the growth conditions of the host-vector systems. As the following data demonstrates supplementing the growth medium for the host-vector system with Cu and/or Zn results in a greater yield of the enzyme in active dimer form.

### Growth of Bacteria Containing pSODβ₁T11

### I. Stock Cultures

Stock cultures of pSODβ₁T₁₁ were grown on casein medium (see inoculum), then diluted twofold with freezing medium and stored at -80°C. Freezing medium contains:

| | |
|---|---|
| K₂HPO₄ | 6.3 gr |
| KH₂PO₄ | 1.8 gr |
| Na Citrate | 0.45 gr |
| MgSO₄·7H₂O | 0.09 gr |
| (NH₄)₂SO₄ | 0.9 gr |
| Glycerol | 44 gr |
| Per 500 ml | |

### II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C, and approximately 200 r.p.m. If needed subsequent stages in inoculum propagation were carried out in stirred aerated fermenters. Sterile medium was inoculated with 2-10% flask culture, and incubated 15 hours at 30°C, pH 7 ± 0.5 with agitation and aeration to maintain dissolved oxygen level above 20% air saturation.

### III. Production

The production medium contains:

| | |
|---|---|
| Casein hydrolysate | 20 g/l |
| Yeast extract | 10 g/l |
| K₂HPO₄ | 2.5 g/l |
| MgSO₄•7H₂O | 1 g/l |
| NaCl | 5 g/l |
| Biotin | 0.1 mg/l |
| Thiamine | 1 mg/l |
| Trace elements solution | 3 ml/l |
| Tetracycline | 12.5 mg/l |

In some of the experiments we added:

| | |
|---|---|
| CuSO₄•5H₂O | 0.8 g/l |
| ZnSO₄•7H₂O | 10 mg/l |

Biotin, thiamine, and tetracycline in concentrated solutions were filter sterilized separately and added to the sterile production medium before inoculation. Sterile glucose solution was added initially to supply 10 g/l. At the induction step another 10 g/l of glucose was added.

The trace elements solution contains:

| | |
|---|---|
| FeCl₃ | 16 g/l |
| ZnCl₂•4H₂O | 2 g/l |
| CoCl₂•6H₂O | 2 g/l |
| Na₂MoO₄•2H₂O | 2 g/l |
| CaCl₂•2H₂O | 1 g/l |
| CuCl₂ | 1 g/l |
| H₃BO₃ | 0.5 g/l |
| Conc. HCl | 100 ml/l |

The medium is inoculated with 0.3 - 10% inoculum culture and incubated at 30°C. Agitation-aeration rates are set to maintain dissolved oxygen level above 20% air saturation. The pH is maintained at 7 ± 0.2 with NH₃. Once cell concentration reaches about 3.5 g/l (OD₆₆₀ = 10) induction is started.

The temperature is raised to 42°C and maintained at 42°C for 1-5 hours. The culture is then chilled, and cells are recovered by centrifugation for enzyme purification.

### RECOVERY OF SOD

One and one-half kilograms of bacterial cells (wet cake) are suspended in 12 liters of 50 mM sodium phosphate (pH 7.8), in a Polytron (Kinematica) blender while controlling the speed to minimize foaming. The homogeneous suspension is continuously passed through a Dynomill cell disrupter KD5 (Willy, A. Bachofen, Basel). The homogeneous suspension of disrupted cells is sonicated using a continuous flow cell and centrifuged in a CEPA 101 centrifuge. The supernatant is heated for 2 hours at 65°C, cooled and centrifuged as before. The clear supernatant is concentrated to 1 liter in a Millipore Pellicon ultrafiltration device using 10,000 molecular weight cutoff casettes (type PTGC). The concentrated protein solution is passed through a DEAE-Sephacel column (2 Kg DEAE Sephacel) equilibrated with 150 mM sodium phosphate buffer (pH 7.8). The flow through solution is collected, concentrated and dialyzed in a Pellicon ultrafiltration device against 20 mM Tris-HCL, pH, 7.8 and then applied on to a QAE-Sepharose column equilibrated with 20 mM Tris-HCl buffer. The column is developed with a 20 mM Tris-HCl buffer, pH 7.8, and a salt gradient (0 - 200 mM NaCl). SOD containing fractions are collected, concentrated using a Pellicon ultrafiltration device, dialyzed against distilled water and then brought to 100 mM sodium acetate by adding 1M sodium acetate buffer, pH 4.8. The protein solution is then further separated on a CM-Sepharose column equilibrated with 100 mM sodium acetate buffer, pH 4.7. The column is developed using the same buffer and a salt gradient (100-500 mM NaCl). SOD containing fractions are collected, concentrated using a Pellicon ultrafiltration device and lyophilized.

### EXAMPLE 10

### Enzymatic Activity of SOD Produced by Bacteria

The purified human SOD (hSOD) produced by bacteria in Example 26 under standard growth conditions (that is without adding extra CuSO₄) possessed only 5% of enzymatic activity as compared to bovine Cu/Zn SOD. Analysis of the metal content reveals that the enzyme contains little Cu, and that is only 8% of the expected value. Furthermore, it seems that most of the Cu⁺⁺ sites are replaced by Zn ions since the protein contains almost twice the Zn required. However, completely metal free apoprotein, prepared from the bacterially produced hSOD, regains essentially full enzymatic activity upon reconstitution in solution. The solution contains both Cu⁺⁺ and Zn⁺⁺, each at a concentration of 1.2 mole ions per mole active site. (Table IV).

The data presented above suggested that the intracellular concentration of Cu⁺⁺ is limited and insufficient to saturate the hSOD produced. In a series of experiments we have demonstrated that elevated Cu⁺⁺ concentrations in the growth medium caused increase in the specific activity of hSOD. Indeed, E. coli grown in casein hydrolysate (Example 26) supplemented with 200 ppm Cu⁺⁺, produced, after induction, fully active hSOD with the natural composition of metals, and full enzymatic activity (Table IV). Additional experiments have demonstrated the same effect when the amount of exogenous Cu⁺⁺ added ranged from 50-250 ppm. We have seen a similar effect with LB (Luria Broth) medium supplemented with 75 ppm Cu⁺⁺.

**TABLE IV**

| Activity and Metal Content of SOD Preparations | | | |
|---|---|---|---|
| | Mole/Subunit | | Activity |
| Protein | Cu | Zn | u/mg |
| hSOD¹ | 0.07 | 1.62 | 167 |
| Apo enzyme | 0.01 | 0.02 | 0 |
| Reconstituted SOD | 0.81 | 0.88 | 2931 |
| hSOD² | 0.88 | 0.90 | 2730 |
| Bovine SOD | 0.97 | 1.01 | 2805 |
| hSOD (Sigma) | | | 1606 |

| | | | |
|---|---|---|---|
| ¹hSOD prepared as described in Example 26. The medium used did not contain the added Cu⁺⁺ and Zn⁺⁺. | | | |
| ²hSOD prepared as described in Example 26. The medium used did contain the added Cu⁺⁺ and Zn⁺⁺. | | | |

SOD concentrations were determined by the method of Lowry (Lowry, O.H., Rosebrough, N.J., Farr, A.L. and Randall, R.J., J. Biol. Chem. 193, 265-275 (1951)) using boving serum albumin as standard. Activity measurement monitoring the inhibition of reduction of ferricytochrome-c were carried out as described by McCord and Fridovich (McCord, J.M. and Fridovich, I., J. Biol. Chem. 244, 6049-6055 (1969)). Cu and Zn content in pure SOD preparations was determined by atomic absorption. SOD-apo enzyme was prepared according to Weser and Hartmann (Weser, U. and Hartmann, H.J., FEBS Lett. 17, 78-80 (1971)) and reconstituted by simultaneous addition of Cu and Zn (Jewett, S.L., Latrenta, G.S. and Beck, C.M., Arc. Biochem. Biophys. 215, 116-128 (1982)).

### EXAMPLE 11

### Amino Terminal Sequence of Bacterial Produced hSOD

The sequence of 5 amino acids at the amino terminus of purified SOD prepared as described in Example 26 was determined by Edmann degradation. The amino acid sequence is identical to the N-terminus of human Cu/Zn SOD, that is Ala-Thr-Lys-Ala-Val. This confirms the authenticity of the bacterial product. It seems therefore that the soluble hSOD is accessible to E. coli processing enzymes which remove the N-terminal methionine.

### Discussion

We have demonstrated that bacteria which are induced to produce large amounts of hSOD when grown on LB or casein hydrolysate media, produce an enzymatically inactive protein. The protein so produced can be activated by reconstituting and adding back the missing Cu⁺⁺, Zn⁺⁺ ions. Unexpectedly, the bacteria can be induced to produce enzymatically active hSOD by growing on media which has been supplemented with Cu⁺⁺. While not wishing to be bound by theory, we believe that certain components of rich media (such as LB and casein hydrolysate), chelate most of the available copper, such that the bacteria do not have available enough free copper to fill all the sites in the SOD molecules, which are being produced at elevated levels. The addition of 50-250 ppm of Cu⁺⁺ ions to the media apparently raises the Cu⁺⁺ concentration within the bacteria to levels which are sufficient to provide all the Cu⁺⁺ necessary for the overproduced human Cu/Zn SOD.

## Claims

1. A method of producing an enzymatically active polypeptide analog of human Cu/Zn superoxide dismutase having an amino acid sequence substantially identical to that of naturally occurring human Cu/Zn superoxide dismutase, and the biological activity of naturally occurring human Cu/Zn superoxide dismutase which comprises:
(a) growing a culture of bacterial cells, transformed with a plasmid containing DNA encoding said polypeptide analog of human Cu-Zn superoxide dismutase incorporated therein, in a zinc-containing production medium supplemented with a non-growth inhibitory amount of Cu⁺⁺ such that the final concentration each of Cu⁺⁺ and Zn⁺⁺ in the medium is greater than 2 ppm, wherein said transformed cells are capable of expressing the DNA encoding said polypeptide analog of human Cu/Zn superoxide dismutase; and
(b) recovering the enzymatically active polypeptide analog of human Cu/Zn superoxide dismutase so produced.

2. A method as in claim 1, wherein the bacterial cells comprise Escherichia coli cells.

3. A method of claim 1 wherein the plasmid is pSODα2 having the restriction map shown in Fig. 1 and deposited under ATCC Accession No. 39786; pSODβ1 having the restriction map shown in Fig. 2 and being 3.0 kb in length; pSODβ₁T₁₁ having the restriction map shown in Fig. 3 and being 3.7 kb in length; pSODβ₁TT-1 having the restriction map shown in Fig. 4 and being 4.8 kb in length, or pSODβ₁-BA2 having the restriction nap shown in Fig. 5.

4. A method as in claim 1, wherein the bacterial cell is Escherichia coli strain A2097 containing plasmid pSODα2 ATCC 39786.

5. A method as in claim 1, wherein the production medium is a casein hydrolysate medium.

6. A method as in claim 5, wherein the casein hydrolysate medium is LB medium.

7. A method as in claim 1, wherein the Cu⁺⁺ concentration is from about 50 to about 250 ppm.

8. A method as in claim 7, wherein the Cu⁺⁺ concentration is about 200 ppm.

9. A method as in claim 7, wherein the Cu⁺⁺ concentration is about 75 ppm.

10. A method as in claim 1, wherein the prcduction medium is also supplemented with an amount of Zn⁺⁺ so that the concentration of Zn⁺⁺ in the medium is greater than about 2 ppm.

11. A method as in claim 10, wherein the production medium is supplemented with about 0.8 g/l CuSO₄.5H₂O and about 10 mg/l ZnSO₄.7H₂O.

12. A method of claim 1 wherein after step (a), the resulting bacterial cell culture is induced to express the DNA and produce the enzymatically active human Cu/Zn superoxide dismutase polypeptide analog.

13. A method as in claim 12, wherein the induction ccmprises heat induction.

14. A method of producing a purified enzymatically active polypeptide analog of human Cu/Zn superoxide dismutase according to claim 1 or 12, wherein the recovery of step (b) comprises:
(a) Isolating the bacterial cells from the production medium;
(b) disrupting the bacterial cells so as to form a suspension comprising cell debris and a protein supernatant solution;
(c) separating said cell debris from the soluble protein supernatant solution;
(d) heating said separated protein supernatant at about 65°C for about 2 hours;
(e) cooling the heated protein supernatant;
(f) separating the resulting protein supernatant so as to produce a clear supernatant protein solution containing the superoxide dismutase analog thereof; and
(g) recovering the enzymatically active human Cu/Zn superoxide dismutase analog from the clear supernatant protein solution.

15. The method of claim 14, wherein recovering the enzymatically active human Cu/Zn superoxide dismutase analog from the clear supernatant protein solution comprises:
(a) treating the clear supernatant protein solution to obtain a concentrated solution of the superoxide dismutase analog;
(b) subjecting the concentrated solution to both anion and cation exchange chromatography; and
(c) collecting the resulting fractions containing purified superoxide dismutase analog thereof.

## Patentansprüche

1. Verfahren zur Herstellung eines enzymatisch aktiven Polypeptidanalogons von Human-Cu/Zn-Superoxiddismutase mit einer zu der von natürlich vorkommender Human-Cu/Zn-Superoxiddismutase im wesentlichen identischen Aminosäuresequenz und der biologischen Aktivität von natürlich vorkommender Human-Cu/Zn-Superoxiddismutase, umfassend:
(a) Wachsenlassen einer Kultur von mit einem Plasmid, das darin eingebaut DNA mit Codierung für das Polypeptidanalogon von Human-Cu/Zn-Superoxiddismutase enthält, transformierten Bakterienzellen in einem zinkhaltigen Produktionsmedium, das mit einer nichtwachstumshemmenden Menge von Cu⁺⁺ derart ergänzt ist, daß die Endkonzentration von jeweils Cu⁺⁺ und Zn⁺⁺ im Medium mehr als 2 ppm beträgt, wobei die transformierten Zellen zur Expression der DNA mit Codierung für das Polypeptidanalogon von Human-Cu/Zn-Superoxiddismutase fähig sind, und
(b) Gewinnen des so hergestellten enzymatisch aktiven Polypeptidanalogons von Human-Cu/Zn-Superoxiddismutase.

2. Verfahren gemäß Anspruch 1, wobei die Bakterienzellen *Escherichia coli*-Zellen umfassen.

3. Verfahren gemäß Anspruch 1, wobei das Plasmid pSODα2 mit der in Fig. 1 angegebenen Restriktionskarte, das unter der ATCC-Hinterlegungsnummer 39786 hinterlegt ist, pSODβ1 mit der in Fig. 2 angegebenen Restriktionskarte und einer Länge von 3,0 kb, pSODβ₁T₁₁ mit der in Fig. 3 angegebenen Restriktionskarte und einer Länge von 3,7 kb, pSODβ₁TT-1 mit der in Fig. 4 angegebenen Restriktionskarte und einer Länge von 4,8 kb oder pSODβ1-BA2 mit der in Fig. 5 angegebenen Restriktionskarte ist.

4. Verfahren gemäß Anspruch 1, wobei die Bakterienzelle vom das Plasmid pSODα2, ATCC 39786, enthaltenden *Escherichia coli*-Stamm A2097 ist.

5. Verfahren gemäß Anspruch 1, wobei das Produktionsmedium aus einem Caseinhydrolysatmedium besteht.

6. Verfahren gemäß Anspruch 5, wobei das Caseinhydrolysatmedium aus LB-Medium besteht.

7. Verfahren gemäß Anspruch 1, wobei die Cu⁺⁺-Konzentration etwa 50 bis etwa 250 ppm beträgt.

8. Verfahren gemäß Anspruch 7, wobei die Cu⁺⁺-Konzentration etwa 200 ppm beträgt.

9. Verfahren gemäß Anspruch 7, wobei die cu⁺⁺-Konzentration etwa 75 ppm beträgt.

10. Verfahren gemäß Anspruch 1, wobei das Produktionsmedium auch so mit einer Zn⁺⁺-Menge ergänzt ist, daß die Konzentration von Zn⁺⁺ im Medium mehr als etwa 2 ppm beträgt.

11. Verfahren gemäß Anspruch 10, wobei das Produktionsmedium mit etwa 0,8 g/l CuSO₄ · 5 H₂O und etwa 10 mg/l ZnSO₄ · 7 H₂O ergänzt ist.

12. Verfahren gemäß Anspruch 1, wobei nach der Stufe (a) die erhaltene Bakterienzellkultur zur Expression der DNA und Erzeugung des enzymatisch aktiven Human-Cu/Zn-Superoxiddismutase-Polypeptidanalogons induziert wird.

13. Verfahren gemäß Anspruch 12, wobei die Induktion eine Wärmeinduktion umfaßt.

14. Verfahren zur Herstellung eines gereinigten, enzymatisch aktiven Polypeptidanalogons von Human-Cu/Zn-Superoxiddismutase gemäß Anspruch 1 oder 12, wobei das Gewinnen von Stufe (b) umfaßt:
(a) Isolieren der Bakterienzellen aus dem Produktionsmedium,
(b) Aufbrechen der Bakterienzellen zur Bildung einer Suspension, die Zellabfall und eine überstehende Proteinlösung umfaßt,
(c) Abtrennen des Zellabfalls von der überstehenden Lösung von löslichem Protein,
(d) Erhitzen des abgetrennten Proteinüberstands während etwa 2 h auf etwa 65 °C,
(e) Abkühlen des erhitzten Proteinüberstands,
(f) Abtrennen des erhaltenen Proteinüberstands zur Herstellung einer klaren überstehenden Proteinlösung, die das Superoxiddismutaseanalogon enthält, und
(g) Gewinnen des enzymatisch aktiven Human-Cu/Zn-Superoxiddismutaseanalogons aus der klaren überstehenden Proteinlösung.

15. Verfahren von Anspruch 14, wobei das Gewinnen des enzymatisch aktiven Human-Cu/Zn-Superoxiddismutaseanalogons aus der klaren überstehenden Proteinlösung folgende Stufen umfaßt:
(a) Behandeln der klaren überstehenden Proteinlösung zur Gewinnung einer konzentrierten Lösung des Superoxiddismutaseanalogons,
(b) Durchführen sowohl einer Anionen- als auch einer Kationenaustauschchromatographie bei der konzentrierten Lösung, und
(c) Sammeln der erhaltenen Fraktionen, die gereinigtes Superoxiddismutaseanalogon enthalten.

## Revendications

1. Procédé de production d'un analogue polypeptidique actif du point de vue enzymatique de la superoxyde-dismutase Cu/Zn humaine ayant une séquence d'acides aminés sensiblement identique à celle de la superoxyde-dismutase Cu/Zn humaine naturelle, et l'activité biologique de la superoxyde-dismutase Cu/Zn humaine naturelle, qui consiste à :
(a) faire croître une culture de cellules bactériennes, transformées avec un plasmide contenant un ADN codant ledit analogue polypeptidique de la superoxyde-dismutase Cu/Zn humaine incorporé dedans, dans un milieu de production contenant du zinc complémenté avec une quantité de Cu⁺⁺, n'inhibant pas la croissance, telle que la concentration finale de chacun de Cu⁺⁺ et Zn⁺⁺ dans le milieu soit supérieure à 2 ppm, dans laquelle lesdites cellules transformées sont capables d'exprimer l'ADN codant ledit analogue polypeptidique de la superoxyde-dismutase Cu/Zn humaine ; et
(b) récupérer l'analogue polypeptidique actif du point de vue enzymatique de la superoxyde-dismutase Cu/Zn humaine ainsi produit.

2. Procédé selon la revendication 1, dans lequel les cellules bactériennes comprennent des cellules Escherichia coli.

3. Procédé selon la revendication 1, dans lequel le plasmide est pSODα2 ayant le schéma de restriction indiqué sur la Figure 1 et déposé sous ATCC Accession N° 39786 ; pSODβ1 ayant le schéma de restriction indiqué sur la Figure 2 et ayant une longueur de 3,0 kb ; pSODβ₁T₁₁ ayant le schéma de restriction indiqué sur la Figure 3 et ayant une longueur de 3,7 kb ; pSODβ₁TT-1 ayant le schéma de restriction indiqué sur la Figure 4 et ayant une longueur de 4,8 kb, ou pSODβ₁-BA2 ayant le schéma de restriction indiqué sur la Figure 5.

4. Procédé selon la revendication 1, dans lequel la cellule bactérienne est la souche Escherichia coli A2097 contenant le plasmide pSODα2 ATCC 39786.

5. Procédé selon la revendication 1, dans lequel le milieu de production est un milieu à base d'hydrolysat de caséine.

6. Procédé selon la revendication 5, dans lequel le milieu à base d'hydrolysat de caséine est un milieu LB.

7. Procédé selon la revendication 1, dans lequel la concentration de Cu⁺⁺ est d'environ 50 à environ 250 ppm.

8. Procédé selon la revendication 7, dans lequel la concentration de Cu⁺⁺ est d'environ 200 ppm.

9. Procédé selon la revendication 7, dans lequel la concentration de Cu⁺⁺ est d'environ 75 ppm.

10. Procédé selon la revendication 1, dans lequel le milieu de production est aussi complémenté avec une quantité de Zn⁺⁺ telle que la concentration de Zn⁺⁺ dans le milieu soit supérieure à environ 2 ppm.

11. Procédé selon la revendication 10, dans lequel le milieu de production est complémenté avec environ 0,8 g/l de CuSO₄·5H₂O et environ 10 mg/l de ZnSO₄·7H₂O.

12. Procédé selon la revendication 1, dans lequel, après l'étape (a), la culture de cellules bactériennes résultante est induite de façon à exprimer l'ADN et produire l'analogue polypeptidique de la superoxyde-dismutase Cu/Zn humaine actif du point de vue enzymatique.

13. Procédé selon la revendication 12, dans lequel l'induction comprend une induction thermique.

14. Procédé de production d'un analogue polypeptidique actif du point de vue enzymatique purifié de superoxyde-dismutase Cu/Zn humaine selon la revendication 1 ou 12, dans lequel la récupération de l'étape (b) consiste à :
(a) isoler les cellules bactériennes du milieu de production ;
(b) rompre les cellules bactériennes de façon à former une suspension comprenant des débris de cellules et une solution surnageante de protéines ;
(c) séparer lesdits débris de cellules de la solution surnageante de protéines solubles ;
(d) chauffer ledit surnageant de protéines séparé à environ 65°C pendant environ 2 heures ;
(e) refroidir le surnageant de protéines chauffé ;
(f) séparer le surnageant de protéines résultant de façon à produire une solution de protéines surnageante limpide contenant son analogue de superoxyde-dismutase ; et
(g) récupérer l'analogue de la superoxyde-dismutase Cu/Zn humaine actif du point de vue enzymatique à partir de la solution de protéines surnageante limpide.

15. Procédé selon la revendication 14, dans lequel la récupération de l'analogue de la superoxyde-dismutase Cu/Zn humaine actif du point de vue enzymatique à partir de la solution de protéines surnageante limpide consiste à :
(a) traiter la solution de protéines surnageante limpide de façon à obtenir une solution concentrée de l'analogue de superoxyde-dismutase ;
(b) soumettre la solution concentrée à une chromatographie par échange tant d'anions que de cations ; et
(c) recueillir les fractions résultantes contenant son analogue de superoxyde-dismutase purifié.
